# EUROPEAN PATENT APPLICATION

(11) **EP 3 248 604 A1**
(43) Date of publication of application: **29.11.2017**
(21) Application number: 16171765.7
(22) Date of filing: 27.05.2016
(51) Int. Cl.: A61K 31/585, A61K 31/704, A61P 35/00

(54) **AGENTS FOR MODULATION OF WNT-TCF SIGNALLING AND USES THEREOF**

(71) Applicant: UNIVERSITE DE GENEVE, 1211 Geneva 4 (CH)
(72) Inventor: RUIZ ALTABA, Ariel, 1205 GENEVE (CH)
(74) Representative: reuteler & cie SA

(57) **Abstract**

The present invention relates to inhibitors of WNT-TCF signalling and compositions thereof. The invention further relates to use of those inhibitors of WNT-TCF signalling and compositions thereof for the treatment and/or prevention and/or prevention of recurrence of a WNT-activated cancer and/or a cancer associated with the cancer stem cells. the active compounds are certain cardenolides and bufanolides.

## Description

### Field of the Invention

The present invention relates to new agents that are WNT-TCF signalling inhibitors and their use in the prevention, treatment or prevention of recurrence of cancers.

### Background of the Invention

WNT (Wingless) signaling plays an important role in regulating cell proliferation and renewal. WNT signaling can be broadly classified in two pathways: canonical (involving β-catenin/transcription T cell factor/lymphoid enhancer factor (TCF/LEF) interaction) and non-canonical (involving mitogen-activated protein kinases (MAPK) cascade). An active canonical WNT pathway describes a series of events that occur when secreted WNT proteins bind to cell-surface receptors causing an accumulation of β-CATENIN in the cytoplasm, and shuttling β-CATENIN into the nucleus to complex with TCF/LEF complex, which, in turn results in an activation of WNT-TCF transcriptional response (Nusse R., 2008, Cell Res., 18:523-7*).*

Constitutive activation of the canonical WNT-TCF pathway is common in a number of human cancers, such as those of the colon cancer through loss of function mutations *in APC* (adenomatous polyposis coli) gene. Free from APC-dependent degradation nuclear β-CATENIN associates with TCF factors to regulate canonical WNT target genes. Given the roles of WNT-TCF signaling in the control of cancer stem cells and tumor viability, a major effort is underway to find WNT pathway blockers for use in the clinics, and while natural and synthetic small molecules are in development so far none are approved for patient use *(*Anastas et al., 2013, Nat Rev Cancer, 13(1): 11-26*;* Fuentes et al., 2015, Nat Prod Rep., 32(12):1622-8*).*

New antagonists presently in clinical trials block WNT pathway activity at the level of ligand secretion or signal transduction, inhibiting Porcupine *(*Liu et al., 2013, PNAS, 110(50): 20224-9*)* or Tankyrases *(*Huang et al., 2009, Nature, 461(7264): 614-20*;* Chen et al., 2009, Nat Chem Biol, 5(2):100-7*),* respectively. However, many tumors harbor an activated WNT-TCF pathway downstream of these steps. Compounds already approved for human use have also been found to harbor novel activities that block WNT-TCF responses in cancer cells. These notably include two anti-parasitics: pyrvinium and ivermectin. Pyrvinium affects CK1 α kinase activity but has poor bioavailability *(*Thorne et al., 2010, Nat Chem Biol, 6(11):829-36*).* In contrast, it was found that ivermectin *(*Melotti et al., 2014, EMBO Mol Med., 6(10): 1263-78*)* blocks WNT-TCF pathway activity downstream of APC, as it represses TCF targets activated by an APC-insensitive β-catenin form, and could be repositioned as an anti-cancer agent.

Withanolides initially designated a class of natural compounds isolated from plants of the family *Solanaceae* and were structurally characterized by a highly oxygenated A/B-ring system of a steroid skeleton which is based on an ergostane core structure and a δ-lactone side chain E. *(*Glotter, 1991, Nat. Prod. Rep. 8: 415-440*).* This family includes a large number of compounds and over the past 46 years, approximately 750 withanolides exhibiting more than 22 carbon skeleton have been reported from various plant sources *(*Zhang et al., 2012, Pure Appl. Chem., 84(6), 1353-1367*).*
Several withanolides have been found to have cytotoxic effects, such as withanolide D from *Withania somnifera* and withanolide E from *Physalis peruviana* that have been shown to inhibit the cell cycle at G2/M and promote apoptosis of pancreatic cancer cells (Sarkar et al., 2014, Endocr Relat Cancer, 21(1): 113-25*),* and to induce apoptosis through TRAIL *(*Henrich et al., 2015, Cell Death Dis., 6: e1666*),* respectively. Withanolides have been found to have cytotoxic effects and/or affect multiple pathways.
Withanolide F from *Withania adpressa* has been reported to potentially have non-specific cytotoxic activity against cancer cells inducing apoptosis and those cytotoxic effects were less prominent than those of withanolide J or withanolide 14α,15α,17β,20β-tetrahydroxy-1-oxo-(22R)-witha-2,5,24-trienolide *(*Abdeljabbar et al., 2009, Therapie, 64(2): 121- 7*).*
Another withanolide, so-called coagulin-L, originally identified from *Withania coagulans,* has been proposed to positively affect WNT-β-CATENIN signaling as a putative agonist *(*Beg et al., 2014, Phytomedicine, 21(4): 406-14*).*

Cardiac glycosides are also natural compounds isolated from plants and animals. Structurally, the cardiac glycosides are characterized by a steroid skeleton and by the presence of lactone (gamma or delta) side chain and possibly sugar moiety (Newman et al., 2008, Molecular Inteiventions, 8(1), 36-39*).* Cardiac glycosides have been reported to affect Na+/K+ ATPase pump and cardiac functions and hence their therapeutic uses primarily involved the treatment of cardiac failure and cardiac arrhythmia. A variety of isolated cardiac glycoside compounds have been cited for their antiproliferative effect and it was suggested that these may control malignant cell proliferation (*Newman et al., 2008, supra).* In particular, it has been suggested that ouabain may potentiate anoikis and decrease metastases *(*Simpson et al., 2009, Cancer Res, 69(7): 2739-47*).* Cardenolides include digoxigenin, ouabain, digoxin, digitoxin, gitoxigenin and cymarin.
There is an emerging need to find specific inhibitors for blocking pathologically hyperactivated WNT-TCF signaling pathway found in a number of human malignancies for use in the clinics, in particular for the treatment and/or prevention of cancers where cancer cells stemness is an important factor of risk of cancer relapse and metastasis.

### Summary of the Invention

The invention is based on the unexpected findings that the compounds of the invention found to be the inhibitors of the WNT-TCF signaling, are able to block cancer stem cells clonogenicity (self-renewal processes) and induce apoptosis thereof The invention is further based on the observation that repression of the cancer stem cells clonogenicity lasts well beyond the time of compound delivery, possibly by imposing a stable epigenetic change in cancer stem cells, therefore offering a promising tool for cancer treatment, and in particular for prevention of cancer recurrence. The invention is further based on the observation that the compounds of the invention are also able to reduce *in vitro* cancer cells proliferation in multiple cancer cell lines and inhibit *in vivo* tumor growth, possibly by inducing genome-wide transcriptomic changes of WNT-TCF targets. For these reasons, the compounds of the invention are proposed as agents useful for preventing, treating, repressing and/or preventing recurrence of WNT-activated cancers and/or cancers associated with the presence of cancer stem cells, in particular as epidrugs which are known to be useful (e.g. 5-Azacytidine).

One aspect of the invention provides a compound according to the invention, as well as tautomers, geometrical isomers, optically active forms, pharmaceutically acceptable salts, pharmaceutically active derivative and mixtures thereof for use in the prevention, treatment or prevention of recurrence of a WNT-activated cancer and/or a cancer associated with the cancer stem cells.

Another aspect of the invention provides a use of at least one compound according to the invention or a pharmaceutical composition thereof for the preparation of a medicament for the prevention, treatment or prevention of recurrence of a WNT-activated cancer and/or a cancer associated with the cancer stem cells.

Another aspect of the invention provides a pharmaceutical formulation comprising a compound of the invention, or a tautomer, a geometrical isomer, an optically active form, a pharmaceutically acceptable salt, a pharmaceutically active derivative thereof or a mixture thereof, further combined with at least one co-agent useful in the treatment of a cancer, and at least one pharmaceutically acceptable carrier.
Another aspect of the invention provides a method of preventing, treating, repressing or preventing recurrence of a WNT-activated cancer or a cancer associated with cancer stem cells, said method comprising administering to a subject in need thereof at least one compound according to the invention or a pharmaceutical composition thereof.
Another aspect of the invention provides a method of preventing cancer cell proliferation, said method comprising administering to a subject in need thereof at least one compound according to the invention or a pharmaceutical composition thereof.
Another aspect of the invention provides a method of preventing recurrence of cancer, said method comprising administering to a subject in need thereof at least one compound according to the invention or a pharmaceutical composition thereof that induces cancer stem cells apoptosis and/or prevents cancer stem cells clonogenicity or a pharmaceutical composition thereof.
Another aspect of the invention provides a method of down-regulating or inhibiting WNT-TCF signaling and/or inducting stable epigenetic change in cancer cells, said method comprising contacting a cell or a tissue from a patient suffering from a cancer and/or at risk of suffering from recurrence of a cancer with at least one compound according to the invention or a pharmaceutical composition thereof.

### Description of the figures

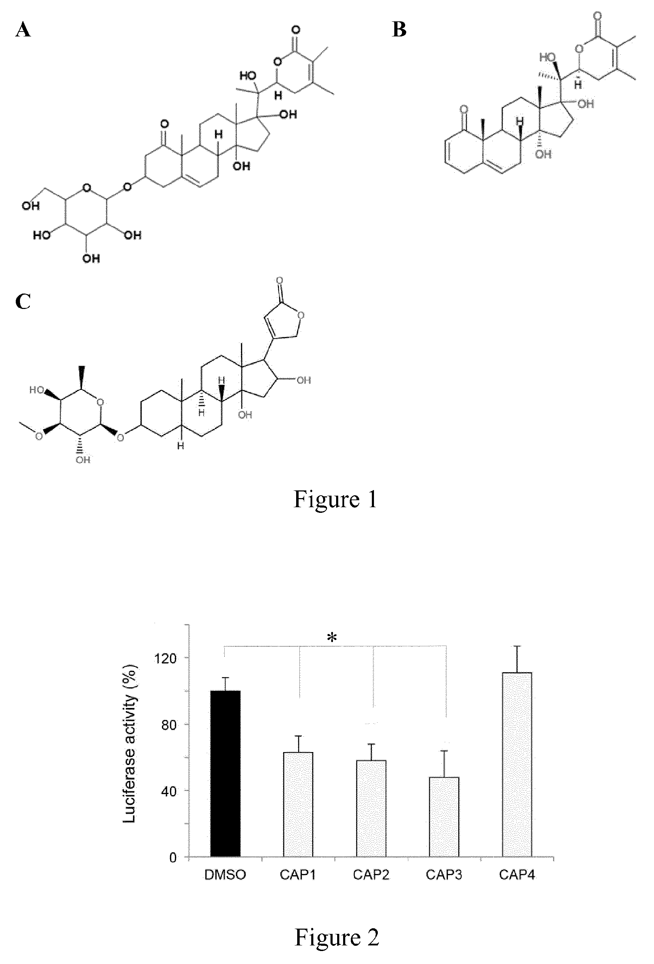

**Figure 1** shows the chemical structures of compounds of the invention. **A**: CAP1; **B**: CAP2; **C:** CAP3.
**Figure 2** shows modulation of WNT-TCF signaling by a dual TCF luciferase activity assay for 293T cells as described in Example 1 represented by the percentage of Luciferase activity after treatment with compounds of the invention CAP1, 2 or 3 as compared to control (DMSO carrier control or CAP4) at 10µM for 12h, *p<0.05.

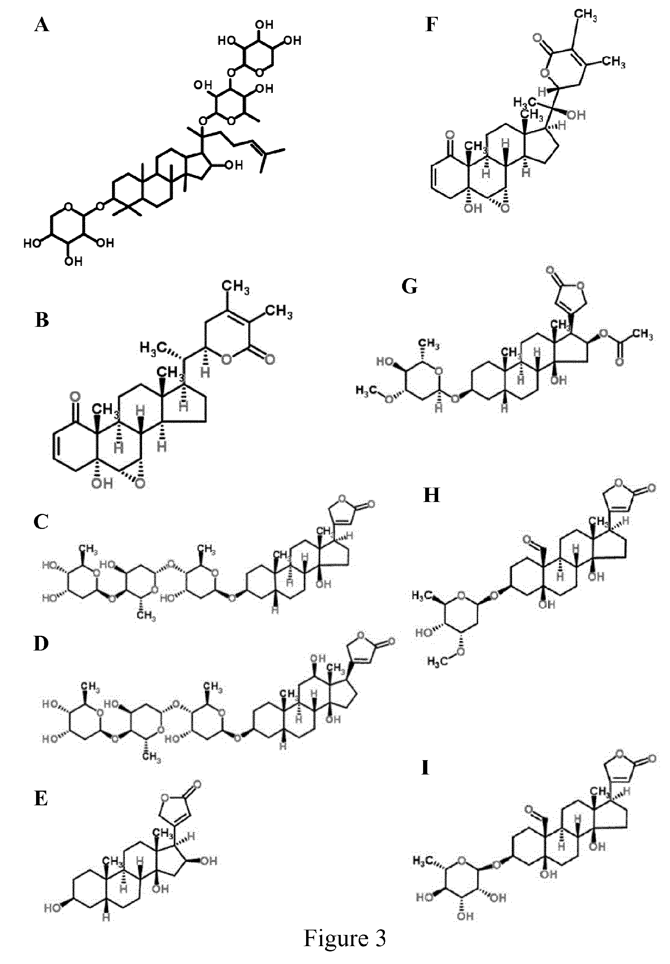

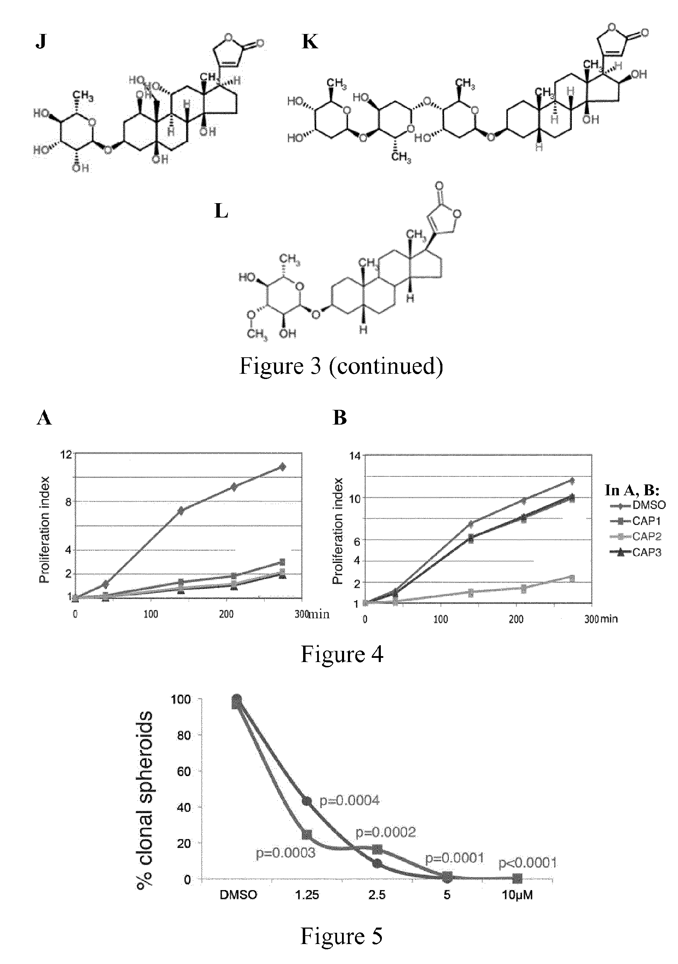

**Figure 3** shows the chemical structure of comparative compounds. **A:** CAP4, **B**: withanolide B, **C:** digitoxin, **D:** digoxin, **E:** gitoxigenin, **F:** withanolide A, **G:** oleandrin, **H:** cymarin, **I**: convallatoxin, **J:** ouabain **K:** gitoxin and **L:** neriifolin.
**Figure 4** shows anti- proliferative properties of CAP1, CAP2 and CAP3 represented by a proliferation index described in Example 2, corresponding to the increase of the CC14 cell number compared to starting amount (equated to 1) over time in minutes; p values are given for the end points in relation to DMSO-treated controls. **A**: At 2.5µM; for CAP2 and CAP3 p=0.0005, for CAP1 p=0.0008. **B**: At 150 nM; for CAP2 p=0.005, for CAP and CAP3 p=0.5.
**Figure 5** shows CAP2-induced block of colon cancer stem cells clonogenicity measured by the inhibition of 3D clonal spheroid formation as described in Example 3. DLD1 (dots) or Ls174T (squares) cells were previously treated with CAP2 at different concentration (x axis) in 2D cultures. Three 96-well plates were used per time point. P values are given in relation to DMSO-treated controls.

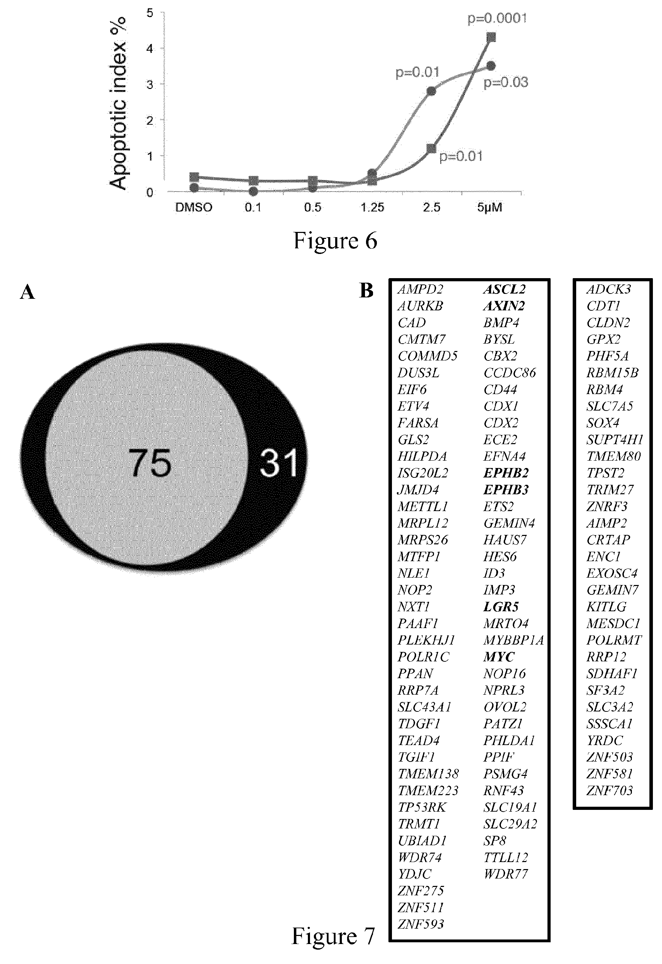

**Figure 6** shows CAP2- induced apoptosis represented by the apoptotic index as described in Example 3 corresponding to the number of cells expressing activated Caspase 3 over total live cell number after treatment with different concentrations (x axis) of CAP2 compared to control (DMSO) in DLD1 (dots) or Ls174T (squares) cells. P values are given in relation to DMSO-treated controls.
**Figure 7** shows the overlap of the 106 TCF1/4-responsive genes from Ls174T colon cancer cells as described in *van der* Flier et al., 2007, Gastroenterology, 132(2): 628-32*)* with the set of genes repressed by CAP2 (75 genes). **A:** Venn diagram representation of the overlap. **B**: Lists of genes - in grey box genes repressed by dnTCF4 and CAP2, in black box unaffected by CAP2 at the dose used but repressed by dnTCF.

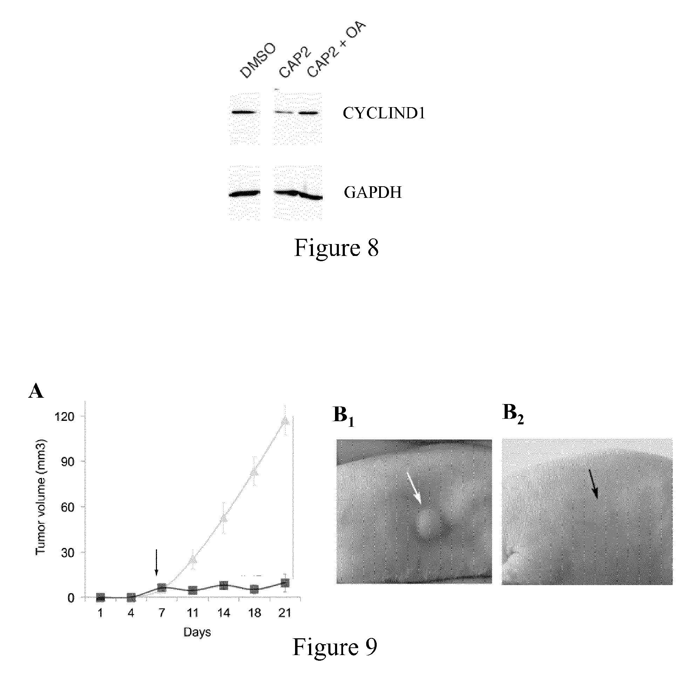

**Figure 8** represents Western blot analyses of the levels of CYCLIN D1 and GAPDH (used as loading control) in DLD1 cells treated with 5 µM CAP2 +/- 15 nM OA compared to cells treated with DMSO (control).
**Figure 9** shows the inhibition of tumor growth after CAP2 treatment in DLD1 xenografts in nude mice from Example 8. **A:** Evolution of tumor volume in time before and after treatment at day 7 (arrow) in control treated group (cyclodextrin alone, triangles, n= 12) compared to cyclodextrin-conjugated CAP2 treated group (squares, n=12). p=0.001 for the endpoint of the experiment. **B_{1,2}**: Representative images of subcutaneous DLD1 xenograft tumor (arrows) growth in control mice **(B₁)** and those receiving CAP2 systemically administered *via* IP (**B₂**).

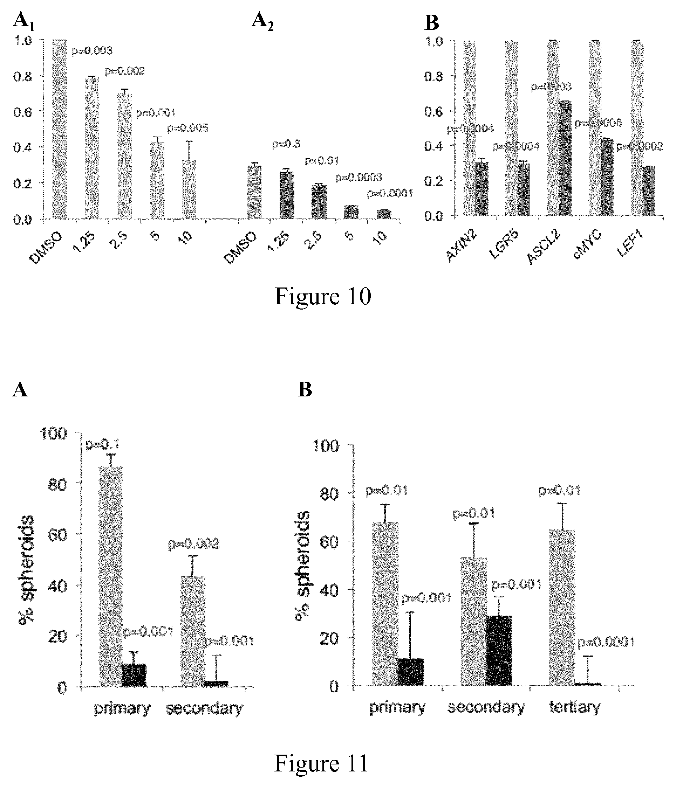

**Figure 10** shows long-lasting repression of WNT-TCF targets by CAP2 assayed as described in Example 9. **A_{1,2}**: Repression of *LGR5* mRNA levels (expressed as a comparison values in comparison to value of expression of DMSO-treated cells from control tumors set to 1) by CAP2 treatment in cells derived from control tumors (**A₁**) and those previously treated with CAP2 (**A₂**), both after dissociation and *in nitro* treatment for 16h with different concentrations of CAP2 (noted on x axis in [µM]) compared to control (DMSO). P values are in relation to the respective DMSO-treated controls. **B**: Normalized gene expression levels in dissociated cells after CAP2-treatment as above but equating the level of CAP2 repression in cells derived from DMSO-treated tumors *in vivo* to 1 (light grey), and comparing this to the level obtained with cells derived from CAP2-treated tumors *in vivo,* re-treated *in vitro* (dark grey). P values are pairwise as shown.
**Figure 11** shows long-lasting repression of 3D clonal spheroids formation after CAP2 treatment (0.5µM: light grey; 2.5µM: dark grey) of 2D cell cultures assayed as described in Example 9 **A:** Percentage of 3D clonal spheroids in primary or secondary assays from DLD1 cells. Spheroids in A and B were not treated with CAP2. **B**: Percentage of 3D clonal spheroids in primary, secondary or tertiary assays from Ls174T cells previously treated in 2D culture with CAP2. Value of DMSO-pretreated control cells is equated to 1. p values in relation to controls.

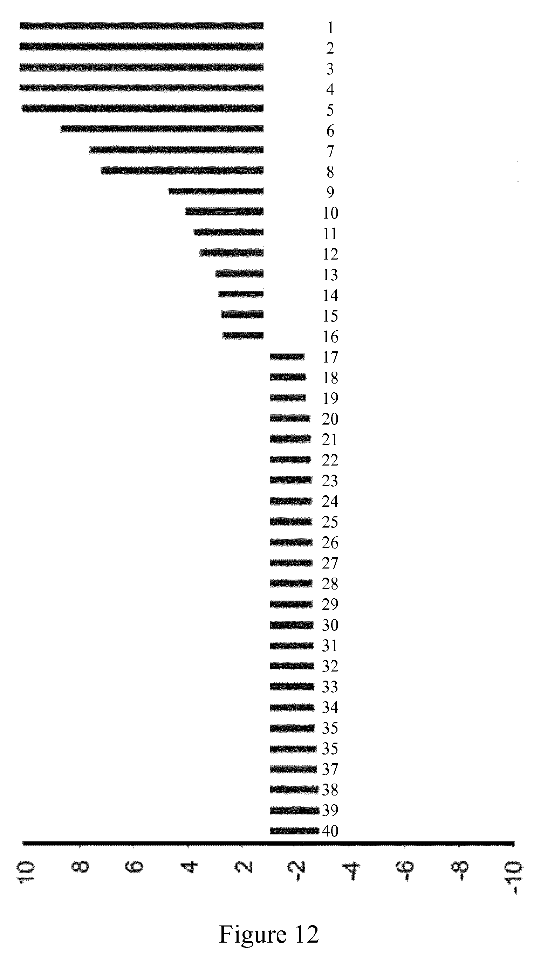

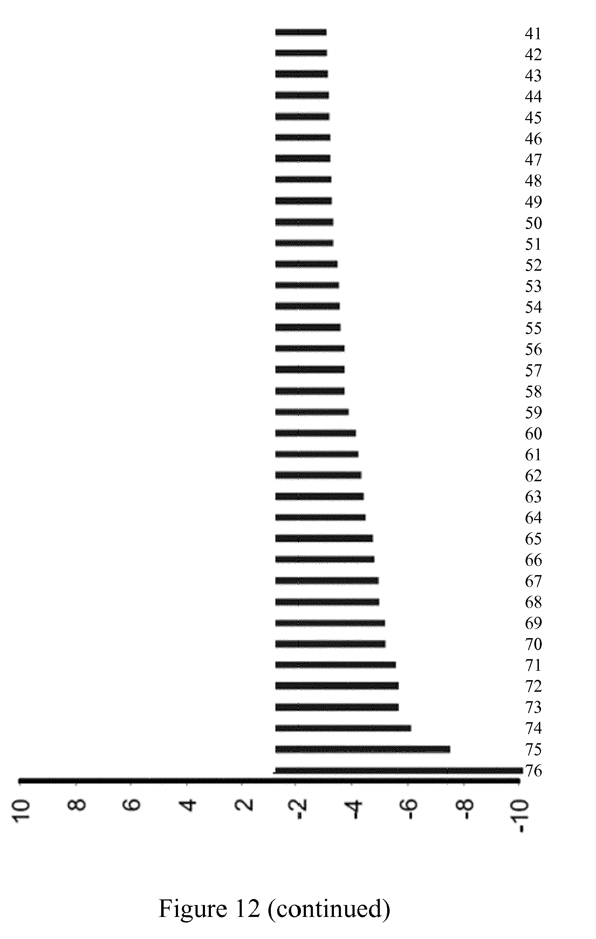

**Figure 12** shows transcriptomic changes imposed by CAP2 on an epigenetic gene set that reveals large modulation (repression or activation) of chromatin remodelers and components. Results of genomic analysis, x axis denotes the genes and corresponding probes from the microarray assay (reliable _a or _a_at probes only) with the associated fold change in relation with DMSO-treated controls for CAP2. List of genes: 1- *HIST1H2BD,* 2- *HIST1H2AB,* 3-*HIST1H2AG,* 4- *HIST2H2BE,* 5- *HIST1H2B,* 6- *PRDM2,* 7- *HIST1H2BK,* 8- *HIST1H3A, 9-HIST1H2A,* 10- *WDR37,* 11- *TAF3,* 12- *PRMT2,* 13- *USP38,* 14- *WDR53,* 15- *KAT8,* 16-*KMT2A,* 17- *HDAC11*, 18- *EPCI,* 19- *MEAF6,* 20- *WHSC1,* 21- *USP45,* 22- *EPC2, 23-DMAP1,* 24- *WDR48, 25- JAK2,26- USP43, 27- CCDC107, 28- NCOA7;* 29- *CCDC71L,* 30-*TAF1A*, 31- *NSD1,* 32- *ARID5B,* 33- *CCDC22,* 34- *SMARCA2,* 35- *PHF16,* 36- *VPS36, 37-SUPT20H,* 38- *COPS5,39- WDR67, 40- CCDC167, 41- MBD2, 42- NCOA1,* 43- *PHF10*, 44-*CHD2,45-MBD4,46-MECOM,47-SETDB1,* 48- *VPS72,* 49- *TADA1,* 50- *CCDC91,* 51-*NCOR1,* 52- *TAF5,* 53- *PHF20L1,* 54- *YEATS4,* 55- *USP13,* 56- *RCOR3,* 57- *PHF11*, 58-*CCDC169,* 59- *PHF17,* 60- *USP31,* 61- *ATF1,* 62- *WDR12,* 63- *ELP6,* 64- *USP48,* 65-*MTA3,* 66- *USP25,* 67- *HMG20A,* 68- *CCDC50, 69- CSRP2BP,* 70- *SAP30,* 71- *TAF5L, 72-SETD6, 73- TBL1XR1,* 74- *SMARCAL1,* 75- *EED* and *76-ZMYND8.* Genes showing changes by 5-fold or more include *ZYMND8, EED, SMARCAL1, PRDM2* and a number of *HISTONE* genes.

### Detailed description

The terms "cancers" or "tumors" as defined herewith are diseases involving abnormal cell growth with the potential to invade or spread to other parts of the body. Term cancers designate diseases exemplified by, but not limited to, carcinomas (such as breast, prostate, lung, pancreas, and colon cancers), melanomas, sarcomas (such as bone, cartilage, nerve cancer), germ cell tumors (such as seminoma and dysgerminoma) and blastomas. According to a particular aspect, the term cancers further defines diseases characterized by the presence of cancer stem cells, described herein as "cancers associated with cancer stem cells", in particular, brain cancer, breast cancer, colon cancer, ovary cancer, pancreas cancer, prostate cancer, melanoma cancer, multiple myeloma cancer and skin cancer.

According to a particular aspect, cancers associated with a high cancer stem cells component, i.e. characterised by increased amounts of cancer stem cells and/or more invasive cancer stem cells as compared to a standard have a significantly worse clinical outcome. Examples of those cancers are glioblastomas, breast cancers and colon cancers.

The progression of a cancer disease is characterized by system wherein stage 0 describes a *carcinoma in situ,* stage I describes localized cancers, stages II and III describe locally advanced cancers, and stage IV describes cancers that have metastasized or spread to other organs or throughout the body. A cancer may also be designated as "a recurrent cancer" which means that it has appeared again after being in remission or after all visible tumor has been eliminated. The cancer recurrence can either be local, meaning that it appears in the same location as the original, or distant, meaning that it appears in a different part of the body. As used herein, "WNT-activated cancer" defines a cancer characterized by excessive WNT signaling through the canonical pathway. Non-limiting examples of WNT-activated cancer are pancreatic cancer, colon cancer, breast cancer, melanoma, skin cancer, oral cancer, head and neck cancer, lung cancer, gastric cancer, mesothelioma, glioblastoma, medulloblastoma and cervical cancer.

The term "cancer stem cells" (CSCs) as defined herewith are a subset of cancer cells that have the properties similar to stem cells that are the ability to give rise to all cell types found in a particular cancer and self-maintain through the processes of self-renewal (this process is also termed clonogenicity *in vitro* that is a property/ability of stem cells to form floating spheroid clones in define media *in vitro*) and differentiation into multiple cell types. CSCs divide asymmetrically to produce one stem cell, which enables the capacity for self-renewal, and one progenitor cell, which allows them to produce phenotypically diverse cancer cells that constitute tumors. CSCs are tumorigenic (or tumor-forming) and persist in tumors as a distinct population and cause relapse and metastasis by giving rise to new tumors. CSCs self-renewal is regulated and mediated by WNT signaling, therefore these cells are also described herein as "WNT-regulated cancer stem cells".

As used herein, "epigenetic change" refers to changes in DNA caused by external factors and not by changes in the DNA sequence itself, which modulates the transcriptional potential of a cell. Non-limiting examples of mechanisms that produce such changes are DNA methylation, histone modification and gene expression modulation *via* action of repressor proteins. Epigenetic changes may last through cell divisions for the duration of the cell's life, and may also last for multiple generations.

According to a particular embodiment, imposing stable epigenetic change in cancer stem cells such as repression of WNT signaling is believed to be useful in cancer treatment, and in particular for prevention of cancer recurrence since cancer stem cells depend on aberrant WNT signaling (Vermeulen et al, 2010, Nat Cell Biol. 12(5):468-76*).*

As used herein, the term "an inhibitor of WNT-TCF signaling pathway" refers to a compound that inhibits or partially inhibits (decreases) the signal transduction of canonical WNT signaling pathway and therefor modify the cell's gene transcription.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a cancer in a mammal, particularly a human, and includes inhibiting the disease, i.e., arresting its development; or relieving the disease, i.e., causing regression of the disease and/or its symptoms or conditions such as improvement or remediation of damage. In particular, the compounds, methods, uses, formulations and compositions according to the invention are useful in the treatment of cancer and/or in the prevention of evolution of a cancer into an advanced or metastatic stage in patients with early stage cancer, thereby improving the cancer staging and patient prognosis. In particular, prevention and/or treatment of a cancer may include administration of compounds according to the invention.

The term "prevention of cancer recurrence" and the like generally mean obtaining a desired effect of preventing or partially preventing the return of a cancer after the anti-cancer treatment or surgery and after a period of time during which the cancer cannot be detected. The term "efficacy" of a treatment or method according to the invention can be measured based on changes in the course of disease or condition in response to a use or a method according to the invention. According to a particular embodiment, the efficacy can be measured through the assessing of tumor growth, progression and dissemination, the decrease of the number of cancer stem cells or repression of the cancer stem cells clonogenicity.

The term "effective amount" as used herein refers to an amount of at least one compound according to the invention, or a pharmaceutical formulation thereof, that elicits a detectable reduction of the symptoms of the disease in a subject that is being administered said compound, these symptoms can include, for instance decrease in solid tumor mass.

The term "subject" as used herein refers to mammals. For examples, mammals contemplated by the present invention include human, primates, domesticated animals such as cattle, sheep, pigs, horses, laboratory rodents, other pets and the like.

The term "alkyl" when used alone or in combination with other terms, comprises a straight chain or branched C₁-C₆ alkyl which refers to monovalent alkyl groups having 1 to 6 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, and the like. Preferably, these include C₁-C₄ alkyl, which, by analogy, refer respectively to monovalent alkyl groups having 1 to 4 carbon atoms.

The term "alkoxy" refers to the group -O-R where R is "alkyl". Preferred alkoxy groups include for example, methoxy, ethoxy and the like.

### Compounds according to the invention

According to one aspect, is provided a compound of Formula (I): 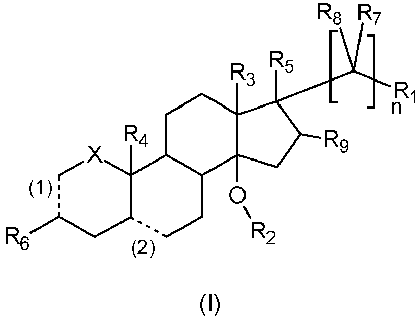 wherein **X** is selected from C=O, -CH and C₁-C₆ alkyl ; **R¹** is an optionally substituted lactone, such as a delta or gamma lactone; **R²** is selected from H and optionally substituted C₁-C₆ alkyl; **R³** and **R⁴** are independently selected from H, OH, optionally substituted C₁-C₆ alkyl such as methyl and optionally substituted alkoxy; **R⁵** and **R⁹** are independently selected from selected from H and OH, wherein one of **R⁵** and **R⁹** is OH; **R⁶** is selected from H and an optionally substituted monosaccharide; **R⁷** and **R⁸** are independently selected from H, OH, optionally substituted C₁-C₆ alkyl such as methyl and optionally substituted alkoxy [; n is an integer selected from 0 and 1; "-----" is selected from a single and a double bond; as well as tautomers, geometrical isomers, optically active forms, pharmaceutically acceptable salts and pharmaceutically active derivative thereof for use in the prevention and/or treatment of a cancer.

According to a particular embodiment, X is CH.

According to another particular embodiment, X is C=O.

According to another particular embodiment, **R¹** is a delta lactone, such as for example a lactone of Formula (II): 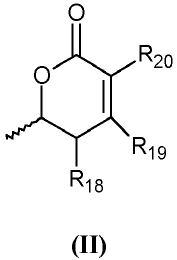 wherein **R¹⁸, R¹⁹** and **R²⁰** are independently selected from H and optionally substituted C₁-C₆ alkyl such as methyl.

According to another particular embodiment, **R¹** is a delta lactone, such as for example a lactone of Formula (III): 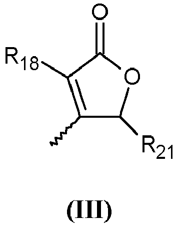 wherein **R¹⁸** and **R²¹** are independently selected from H and optionally substituted C₁-C₆ alkyl such as methyl.

According to a further particular embodiment, **R¹⁸** is H.

According to a further particular embodiment, **R¹⁸** and **R²¹** are H.

According to another further particular embodiment **R¹⁹** and **R²⁰** are methyl.

According to a particular embodiment, n is 1.

According to a particular embodiment, n is 0.

According to a further particular embodiment, n is 1 and **R¹** is a lactone of Formula (II).

According to a further particular embodiment, n is 0 and **R¹** is a lactone of Formula (III).

According to another particular embodiment, **R²** is H.

According to another particular embodiment, the invention **R³** and **R⁴** are optionally substituted C₁-C₆ alkyl such as methyl.

According to another particular embodiment, **R⁵** is OH.

According to another particular embodiment, **R⁹** is OH.

According to another particular embodiment, **R⁶** is H.

According to another particular embodiment, **R⁶** is a monosaccharide of Formula (IV): 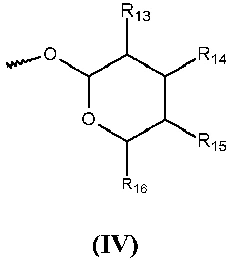 wherein **R¹³**, **R¹⁴, R¹⁵** and **R¹⁶** are independently selected from H, -OR¹⁷ and optionally substituted alkyl such as methyl, wherein **R¹⁷** is selected from H and optionally substituted alkyl, wherein at least one of **R¹³ , R¹⁴, R¹⁵** and **R¹⁶** is -OR¹⁷.

According to a further particular embodiment, R¹⁶ is selected from optionally substituted alkyl such as methyl and -OR¹⁷ wherein **R¹⁷** is optionally substituted alkyl such as methyl. According to another further particular embodiment, R¹³ is OH.

According to another further particular embodiment, R¹⁴ is OH

According to another further particular embodiment, R¹⁵ is OH.

According to another further particular embodiment, R¹³ and R¹⁵ are OH

According to another further particular embodiment, R¹⁵ is OH.

According to another further particular embodiment, R¹⁴ is -OR¹⁷ wherein **R¹⁷** is selected from H and optionally substituted alkyl such as methyl.

According to another particular embodiment, **R⁶** is the following group:

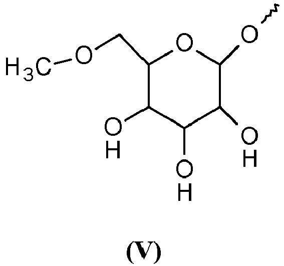

According to another particular embodiment, the invention **R⁶** is the following group:

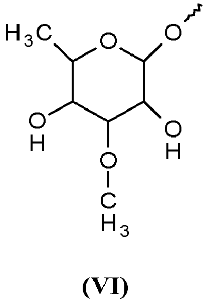

According to another embodiment, compounds are according to Formula (I) wherein bond "----" (1) is a single bond.

According to another embodiment, compounds are according to Formula (I) wherein bond "----" (1) is a double bond.

According to another embodiment, compounds are according to Formula (I) wherein bond "----" (2) is a single bond.

According to another embodiment, compounds are according to Formula (I) wherein bond "----" (2) is a double bond.

According to another embodiment, compounds are according to Formula (I) wherein bonds "-----" (1) and (2) are a single bond.

According to another embodiment, the compounds are according to Formula (I) wherein bonds "-----" (1) and (2) are a double bond.

According to another embodiment, compounds are according to Formula (I) wherein bond "----" (1) is a single bond and bond "-----" (2) is a double bond.

According to a particular embodiment, compounds of the invention re selected from the following group: 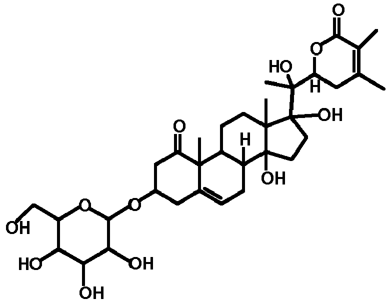 (CAP1, coagulin-L (14*R*,17*S*,20*R*,22*R*)-3β,14,17,20-Tetrahydroxy-1-oxo-witha-5,24-dienolide 3-O-β-D-glucopyranoside); 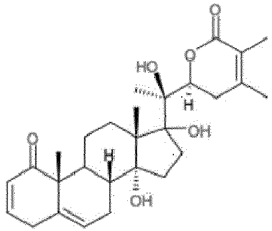 (CAP2, withanolide F, (17α,22R)-14,17,20-Trihydroxy-22,26-epoxyergosta-3,5,24-triene-1,26-dione); and 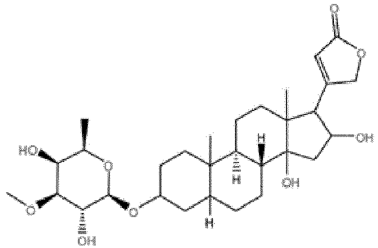 (CAP3, 2,6-dimethyltetrahydropyran-3,4,5-triol; ethane; 3-(3,14,16-trihydroxy-10,13-dimethyl-1,2,3,4,5,6,7,8,9,11,12,15,16,17-tetradecahydro cyclopenta[a]phenanthren-17-yl)-2H-furan-5-one); as well as tautomers, geometrical isomers, optically active forms and pharmaceutically acceptable salts thereof.

According to further embodiment, a compound according to the invention is CAP1.

According to further embodiment, a compound according to the invention is CAP2.

According to further embodiment, a compound according to the invention is CAP3.

According to one embodiment, compounds of the invention may be prepared by synthetic methods.

Alternatively, compounds according to the invention belonging to the group of withanolides can be isolated by standard methods known to the skilled person, for example comprising collecting and drying of plant material and extraction and purification according to known methods as described in Manicassamy, 2014 (PhD thesis, The University of York, The Total Synthesis of Analogues of Withanolide F, http://etheses.whiterose.ac.uk/6373/).

In another embodiment, a compound according to the invention belonging to the group of cardiac glycosides can be isolated by standard methods known to the skilled person, for example comprising collecting and drying of plant material, extraction and purification known methods.

In a particular embodiment, CAP2 can be isolated by standard methods known to the skilled person, for example by collecting and drying aerial parts of *Withania adpressa* followed by a compound extraction with methyl tert-butyl (MTB)-ether/methanol, and isolation by repeated reverse phase chromatography or by a method as described herein.

According to a particular embodiment, compounds of the invention are inhibitors of the WNT-TCF signaling pathway.

### Compositions according to the invention

The invention provides pharmaceutical or therapeutic compounds as compositions and methods for treating a subject, preferably a mammalian subject, and most preferably a human patient who is suffering from a cancer.

Pharmaceutical compositions or formulations according to the invention may be administered as a pharmaceutical formulation, which contains at least one compound of the invention.

The compositions according to the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use by injection or continuous infusion. Injectable compositions are typically based upon injectable sterile saline or phosphate-buffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Compositions of this invention may be liquid formulations including, but not limited to, aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.

In another particular embodiment, compounds of the invention can be formulated as nanoparticles, creams or ointments. Compositions of this invention may be formulated as a depot preparation, which may be administered by implantation or by intramuscular injection. The compounds of this invention can also be administered in sustained release forms or from sustained release drug delivery systems.

According to a particular embodiment, compositions according to the invention are for intravenous use.

According to a particular embodiment, compositions according to the invention are for intra-tumoral use.

According to a particular embodiment, compositions according to the invention are for subcutaneous use.

According to a particular embodiment, compositions according to the invention are for intralymphnodal use.

In another particular aspect, compositions according to the invention are adapted for delivery by single or multiple administrations.

In another particular aspect, compositions according to the invention are adapted for delivery during surgery and after a tumor removal.

According to a particular embodiment, compositions of the invention are veterinary compositions.

Further materials as well as formulation processing techniques and the like are set out in *Part 5 of* Remington's "The Science and Practice of Pharmacy", 22nd Edition, 2012, University of the Sciences in Philadelphia, Lippincott Williams & Wilkins*,* which is incorporated herein by reference.

### Uses according to the invention

According to one aspect of the invention, is provided a use of at least one compound of the invention or a pharmaceutical composition thereof for the treatment and/or prevention of a cancer.

According to a particular aspect of the invention, is provided as use according to the invention wherein the cancer type is a WNT-activated cancer and/or where cancer cells stemness is an important factor of risk of cancer relapse and metastasis.

In another particular embodiment, is provided a use of at least one compound of the invention or a pharmaceutical composition thereof for simultaneously down-regulating expression of WNT-TCF targets *AXIN2, LGR5* in CCs and/or CSCs.

In another particular embodiment, is provided a use of at least one compound of the invention or a pharmaceutical composition thereof for simultaneously down-regulating expression of WNT-TCF targets *AXIN2, LGR5* and *cMYC.*

In another particular embodiment, is provided a use of at least one compound of the invention or a pharmaceutical composition thereof for simultaneously down-regulating expression of WNT-TCF targets *AXIN2, LGR5* and *cMYC* and further up-regulating expression of *CDKN1A* in CCs and/or CSCs.

In another particular embodiment, compounds of the invention are useful in inhibiting epigenetic regulators of cancer cells such as *ZMYND8, EED, SMARCAL1, TBL1XR1, SETD6 TAF5L*.

In another particular embodiment, is provided a use of at least one compound according to the invention or a pharmaceutical composition thereof for preventing CCs and/or CSCs proliferation.

In another particular embodiment, is provided a use of at least one compound according to the invention or a pharmaceutical composition thereof for prevention of CSCs clonogenicity.

In another particular embodiment, is provided a use of at least one compound according to the invention or a pharmaceutical composition thereof for inducing CSCs apoptosis.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

According to another embodiment, the invention provides a method of preventing, treating, repressing or preventing recurrence of a cancer, in particular a WNT-activated cancer or a cancer associated with cancer stem cells, said method comprising administering to a subject in need thereof at least one compound according to the invention or a pharmaceutical composition thereof.

According to one aspect, is provided a method according to the invention for preventing recurrence of a cancer, said method comprising administering to a subject in need thereof at least one compound according to the invention or a pharmaceutical composition thereof that induces cancer stem cells apoptosis and/or prevents cancer stem cells clonogenicity or a pharmaceutical composition thereof.

Another aspect of the invention provides a method of inducing CCs and/or CSCs apoptosis. Another aspect of the invention provides a method according to the invention comprising contacting a cell or a tissue from a patient suffering from a cancer and/or at risk of suffering from recurrence of a cancer with at least one compound according to the invention or a pharmaceutical composition thereof.

According to a particular aspect of the invention, compounds of the invention are useful for modulating WNT-TCF signaling, in particular down-regulating or inhibiting WNT-TCF signalling, notably in the treatment or in the prevention of recurrence of WNT-activated and cancer stem cells sternness associated disorders.

According to another particular aspect of the invention, compounds of the invention are useful for down-regulating WNT-TCF signalling, in particular downstream of APC/βCATENIN complex in particular for inducing simultaneous down-regulation of expression of WNT-TCF targets *AXIN2, LGR5, cMYC* and up-regulation of expression of *CDKN1A* in CCs and/or CSCs. According to a particular aspect those effects persist, from about day 1 to about 3 months after the termination of administration of at least one compound of the invention or a pharmaceutical composition thereof.

According to another particular aspect of the invention, compounds of the invention are useful for inducing of stable epigenetic change in CCs and/or CSCs, such as modulation of chromatin remodelers such as *HISTIH2BD, HISTIH2AB, HISTIH2AG, HIST2H2BE, HISTIH2B, PRDM2, TAF5L, SETD6, TBL1XR1, SMARCAL1, EED* and *-ZMYND8and*/*or* modulation of WNT-TCF targets such as *LGR5, AXIN2*.

### Mode of administration

Compounds and formulations thereof according to this invention may be administered in any manner including orally, parenterally, intravenously, rectally, or combinations thereof. Parenteral administration includes, but is not limited to, intravenous, intra-tumoral, intralymphnodal, intra-arterial, intra-peritoneal, subcutaneous and intramuscular. The compositions of this invention may also be administered in the form of an implant, which allows slow release of the compositions as well as a slow controlled i.v. infusion. Compounds and formulations thereof according to this invention may be administered to a subject in need thereof prior to, simultaneously or sequentially with other therapeutic regimens, and in particular a surgical tumor removal or radiation.

According to another embodiment of the invention, the compounds according to the invention and pharmaceutical formulations thereof can be administered after surgery where solid tumors have been removed as a prophylaxis against relapsing and/or metastases.

Compounds and formulations thereof according to this invention may be administered to a subject in need thereof as a single or as a repeated administration.

### Combination

According to the invention, the compounds according to the invention, and pharmaceutical formulations thereof, can be administered alone or in combination with a co-agent useful in the prevention and/or treatment of a cancer and and/or preventing cancer relapsing, in a therapeutically effective amount such as substances used in conventional chemotherapy, radiation, targeted therapy (e.g. various tyrosine-kinase inhibitors, imatinib mesylate, gefitinib, bortezomib etc.), immunotherapy (e.g. alemtuzumab, ipilimumab, nivolumab etc.), hormonal therapy (e.g. tamoxifen, anastrozole, octreotide etc.) and angiogenesis inhibition (e.g. bevacizumab, thalidomide etc.) directed against cancers.

According to the invention, the compounds according to the invention, and pharmaceutical formulations thereof, can be administered alone or in combination with a co-agent useful in control of establishment of metastases or any other molecule that acts by triggering programmed cell death, differentiation or permanent senescence e.g. for example a co-agent selected from an agent that modulates the hedgehog (Hh)-GLI signaling pathway such as cyclopamine (Clement et al., 2007, Current Biol., 17, 165-172*),* an antimitotic agent such as Cisplatin *(*Petrelli et al, 1989, Cancer Chemother. Pharmacol., 23, 57-6*),* an alkylating agent such as Temozolomide (*Clement et al., 2007, supra*) and anti-Notch *(*Reedijk et al., 2008, Int. J. Oncol., 33, 1223-9*),* anti-TGFβ molecules (Arteaga et al., 2006, Current Opinion in Genetics & Development, 16:30-37).

The invention encompasses the administration of the compounds according to the invention, pharmaceutical formulations thereof, or composition according to the invention, wherein said compounds or compositions are administered to an individual prior to, simultaneously or sequentially with other therapeutic regimens, co-agents useful in the prevention and/or treatment of a cancer, in a therapeutically effective amount.

Compounds or composition according to the invention, or the pharmaceutical formulation thereof, that are administered simultaneously with said co-agents can be administered in the same or different composition(s) and by the same or different route(s) of administration.

According to one embodiment, is provided a pharmaceutical formulation comprising at least one compound of the invention, combined with at least one co-agent useful for preventing, treating, repressing or preventing recurrence of a cancer, and at least one pharmaceutically acceptable carrier.

### Patients

In an embodiment, subjects according to the invention are suffering from a cancer.

In another embodiment, subjects according to the invention are suffering from WNT-activated cancer, or a cancer associated with cancer stem cells, in particular a cancer associated with cancer stem cell component leading to poor prognosis.

In a particular embodiment, subjects according to the invention are suffering from brain cancer, breast cancer, colon cancer, ovary cancer, pancreas cancer, prostate cancer, melanoma cancer, multiple myeloma cancer, skin cancer, oral and neck cancer, head and neck cancer, lung cancer, gastric cancer, mesothelioma, glioblastoma, medulloblastoma or cervical cancer. In another particular embodiment, patients according to the invention are suffering from colon cancer.

In another particular embodiment, patients according to the invention are suffering from lung cancer.

In another particular embodiment, patients according to the invention are suffering from melanoma.

In another particular embodiment, patients according to the invention are suffering from glioblastoma.

In another particular embodiment, patients according to the invention are suffering from basal cell carcinoma.

In another particular embodiment, patients according to the invention are suffering from ovarian cancer.

In one embodiment, subjects according to the invention are subjects suffering from cancer at stage 0/I, or stage II/III, or stage IV.

In one particular embodiment, subjects according to the invention are in cancer remission.

References cited herein are hereby incorporated by reference in their entirety. The present invention is not to be limited in scope by the specific embodiments and drawings described herein, which are intended as single illustrations of individual aspects of the invention, and functionally equivalent methods and components are within the scope of the invention.

### EXAMPLES

The following abbreviations refer respectively to the definitions below:
**293T cells** (human embryonic kidney cells); **APC** (adenomatous polyposis coli); ***ASCL2*** (gene encoding achaete-scute complex homolog 2 transcription factor); ***AXIN2*** (gene encoding a protein axis inhibition protein 2); ***BMP4*** (gene encoding bone morphogenetic protein 4); **BrdU** (5-bromo-2'-deoxyuridine); **CC14** (primary colon cancer cells from bowel tumors); **CC36** (primary colon cancer cells from bowel tumors); ***CDKN1A*** or ***p21*** (gene encoding a cyclin-dependent kinase inhibitor that inhibits the complexes of CDK2 and CDK1); ***cMYC*** (gene encoding a transcription factor); ***CXCL1*** (gene encoding chemokine (C-X-C motif) ligand 1); ***CXCL3*** (gene encoding chemokine (C-X-C motif) ligand 3); ***CXCL5*** (gene encoding chemokine (C-X-C motif) ligand 5); ***DKK1*** (gene encoding Dickkopf WNT signaling pathway inhibitor 1); **DLD1** (colon cancer cell line); **DMEM-F12** (1:1 mix of Dulbecco's modified Eagle medium and Ham's F-12 medium); **dnTCF4** (dominant-negative transcription T cell factor 4); ***EED*** (gene encoding polycomb protein EED);; ***EPHB2*** (gene encoding ephrin receptor B2); ***EPHB3*** (gene encoding ephrin receptor B3); **FBS** (fetal bovine serum); **H358** (human lung cancer cells); **HT29** (human colon cancer cells); ***ID2*** (gene encoding DNA-binding protein inhibitor ID-2); *IL8* (gene encoding interleukin 8); ***IL33*** (gene encoding interleukin 33); ***LEF1*** (gene encoding lymphoid enhancer-binding factor 1); ***LGR5*** (gene encoding encodes leucine-rich repeat-containing G-protein coupled receptor 5); **Ls174T** (human colon cancer cells); **mCCll** (primary colon cancer cells from a liver metastasis); **MeWo** (human melanoma cells); **N**'**ΔβCATENIN** (N-terminally truncated, APC-insensitive βCATENIN); **OA** (okadaic acid); **PBS** (phosphate-buffered saline); **PFA** (paraformaldehyde); ***SETD6*** (gene encoding SET domain containing 6 protein); ***SMAD6*** (gene encoding **[please provide]); *SMARCAL1*** (gene encoding SWI/SNF-related matrix-associated actin-dependent regulator of chromatin subfamily A-like protein 1); ***TAF5L*** (gene encoding TAF5-like RNA polymerase II p300/CBP-associated factor); ***TBL1XR1*** (gene encoding F-box-like/WD repeat-containing protein TBL1XR1); **TCF^{VP16}** (a fusion between TCF and the potent transactivation domain of the viral VP16 protein); **U87** (human glioblastoma cells); **U251** (human glioblastoma cells); ***ZMYND8*** (gene encoding protein kinase C-binding protein 1).

### Statistics

All p given values are from two-tailed student t-tests using triplicates or more as noted.

### Example 1: Identification of specific WNT-TCF response inhibitors

Specific WNT-TCF response inhibitors were identified by using a combination of tests that included performing TCF-binding site driving Firefly luciferase reporter assay, testing for compound's inhibitory effect on cell proliferation and compound's transcriptional modulation of a restricted TCF gene signature.

### Compounds source, extraction and purification

Aerial parts of *Withania adpressa* were collected by Sahara Exporters sarl, (Errachidia, Morocco) in Morocco. Dried plant material was extracted with Methyl tert-butyl (MTB)-ether-methanol. Withanolide F (CAP2) was isolated by repeated reverse phase chromatography. The structure of Withanolide F was confirmed by comparison of NMR and MS data with data reported in the literature *(Abdeljabbar et al., 2007, supra*). Four batches of CAP2 were tested. All tested compound were dissolved in DMSO, except digoxin which was solubilized in MeOH.

The structure of the compounds of the invention CAP1, CAP2 and CAP3 are reported on Figure 1 and those of the comparative compounds on Figure 3.

### Small molecule screens

The primary and secondary screens were performed as described in *Melotti et al., 2014, supra*). Briefly, 293T cells (human embryonic kidney cells) were transfected with TOP Firefly and Renilla luciferase plasmids using the standard CaCl₂ protocol with 5000 cells/well in 96 well plate formats for screening. Cells were treated with compounds for 16h washed with phosphate-buffered saline (PBS) and lysed with passive lysis buffer (Promega). Protein lysates luciferase readouts used Promega Dual Reporter luciferase kit.

### Cells and cultures conditions

Colon cancer cell line LS174T and 293T cells were cultured in DMEM-F12 (1:1 mix of Dulbecco's modified Eagle medium and Ham's F-12 medium with 10% fetal bovine serum (FBS) under standard *in vitro* conditions (37°C, 5% CO₂).

### BrdU incorporation assay

Cells incubated *in vitro* with different compounds were treated in DMEM-F12 with reduced serum (2.5% FBS) for 20 min with BrdU (5-bromo-2'-deoxyuridine; 10mg/ml, Sigma), washed and fixed with fresh paraformaldehyde (PFA; 4%, pH 8) followed by acid treatment and neutralization *(Melotti et al., 2014, supra*). Antibody analyses used anti-BrdU antibodies (supplied by University of Iowa Hybridoma Bank) and rhodamine-coupled anti-mouse secondary antibodies (Invitrogen Molecular Probes). Nuclei were counterstained with DAPI (4',6-diamidino-2-phenylindole; Sigma).

### Microarrays

Cells treated with compounds were collected in Trizol and RNA was harvested using standard protocols. Quality and quantity of RNA was checked using Nano Series (Agilent Technologies). Samples with a RNA Integrity Number (RIN) >9/10 were chosen and cDNA and cRNA samples were prepared using GeneChip IVT Express kit. Genechip hybridization was done using the same kit and applied to PrimeView U219 human array (Affymetrix) in the Genomic Platform at the Faculty of Medicine, University of Geneva.

### Real-time PCR analysis

RT-qPCR was performed as in *Melotti et al. (2014, supra*) using BioRad equipment and reagents. Cells cultured *in nitro* were treated with the desired concentration of drug in DMEM-F12 and reduced serum (2.5% FBS) for 16h, unless otherwise noted, and collected in Trizol (Sigma) before total RNA extraction and cDNA synthesis. Primers were as in *Table 1. HPRT* (gene encoding Hypoxanthine phosphoribosyltransferase 1) and *βACTIN* were used as housekeeping controls for normalization.

**Table 1**

| **Forward (Fw) PCR primers** | **Sequence** | **Reverse (Rev) PCR primers** | **Sequence** |
|---|---|---|---|
| AXIN2 Fw SEQ ID NO.: 1 | CTCCTTATCGTGTGGGC AGT | AXIN2 Rev SEQ ID NO.: 2 | CCAACTCCAGCTTCA GCTTT |
| ASCL2 Fw SEQ ID NO.: 3 | GCGTTCCGCCTACTCGT | ASCL2 Rev SEQ ID NO.: 4 | GGCTTCCGGGGCTGA G |
| LEF1 Fw SEQ ID NO.: 5 | AACCTCTCAGGAGCCCT ACC | LEF1 Rev SEQ ID NO.: 6 | CACGGGCACTTTATTT GAT |
| LGR5 Fw SEQ ID NO.: 7 | GGAGCATTCACTGGCCT TTA | LGR5 Rev SEQ ID NO.: 8 | CTGGACGGGGATTTC TGTTA |
| cMYC Fw SEQ ID NO.: 9 | TGGTCTTCCCCTACCCT CT | cMYC Rev SEQ ID NO.: 10 | GATCCAGACTCTGAC CTTTT |
| CDKN1A/p2 Fw SEQ ID NO.: 11 | GACTCTCAGGGTCGAAA ACG | CDKN1A/p21 Rev SEQ ID NO.: 12 | AAGATGTAGAGCGGG CCTTT |
| EPHB2 Fw SEQ ID NO.: 13 | ATGCGGAAGAGGTGGA TGTA | EPHB2 Rev SEQ ID NO.: 14 | CCTTGAAAGTCCCAG ATGGA |
| EPHB3 Fw SEQ ID NO.: 15 | TGCCACTCAAGCTCTAC TGC | EPHB3 Rev SEQ ID NO.: 16 | GTTATTGTGGCAGGT GCAGA |
| SOX4 Fw SEQ ID NO.: 17 | AAACCAACAATGCCGA GAAC | SOX4 Rev SEQ ID NO.: 18 | GTTCATGGGTCGCTT GATGT |
| ZNRF3 Fw SEQ ID NO.: 19 | CGGGTCATCCCCTGTAC TC | ZNRF3 Rev SEQ ID NO.: 20 | ACGTGAGAGGTTGCT GGTCT |
| KITLG Fw SEQ ID NO.: 21 | TTCCATCATAGACAAAC TTGTGA | KITLG Rev SEQ ID NO.: 22 | GTAAAGAGCCTGGGT TCTGG |
| CXCL3 Fw SEQ ID NO.: 23 | CGTCCGTGGTCACTGAA CT | CXCL3 Rev SEQ ID NO.: 24 | ATGACTTCGGTTTGG GCG |
| IL33 Fw SEQ ID NO.: 25 | TGAGTCTCAACACCCCT CAA | IL33 Rev SEQ ID NO.: 26 | GTTGGCATGCAACCA GAAGT |
| ID3 Fw SEQ ID NO.: 27 | ACCTTCCCATCCAGACA GC | ID3 Rev SEQ ID NO.: 28 | CTTCCGGCAGGAGAG GTT |
| BMP4 Fw SEQ ID NO.: 29 | GATCCACAGCACTGGTC TTG | BMP4 Rev SEQ ID NO.: 30 | GGGATGCTGCTGAGG TTAAA |
| ZMYND8 Fw SEQ ID NO.: 31 | CGAGACCCAGAGTAAA GCCAT | ZMYND8 Rev SEQ ID NO.: 32 | GATGTATCCGCATAG TCAGGG |
| EED Fw SEQ ID NO.: 33 | GTGACGAGAACAGCAA TCCAG | EED Rev SEQ ID NO.: 34 | TATCAGGGCGTTCAG TGTTTG |
| SMARCAL1 Fw SEQ ID NO.: 35 | ACAGCATCAGAGGACT AGCTC | SMARCAL1 Rev SEQ ID NO.: 36 | CACTGGCTTACAAGA CTCCCT |
| TBL1XR1 Fw SEQ ID NO.: 37 | CCAGTTCCCATTTCCTG CCA | TBL1XR1 Rev SEQ ID NO.: 38 | ACACCACAAAAGGAG GCACTT |
| SETD6 Fw SEQ ID NO.: 39 | GGAGGGAACAGCAAGC CTTA | SETD6 Rev SEQ ID NO.: 40 | TCAGGGAACAGGGAA ACTGC |
| TAF5L Fw SEQ ID NO.: 41 | CAGGCAGAACCCCAGC AATA | TAF5L Rev SEQ ID NO.: 42 | CACTGTGCTCTTCGG ACTGT |

To identify WNT-TCF response inhibitors the classic TCF-binding site driving Firefly luciferase reporter assay in human 293T cells was adapted *(*Barker and Clevers, 2006, Nat Rev Drug Discov., 5(12): 997-1014*)* by introducing at every step a genetic benchmark, dominant-negative (dn) TCF4, the effect of which should be mimicked by any antagonist candidate *(Melotti et al., 2014, supra*). In this experimental setting, the expression of N-terminally truncated, APC-insensitive βCATENIN (N'ΔβCATENIN), and of N'βΔCATENIN plus dnTCF4, gave a 5-fold dynamic range for the TCF-binding site-luciferase screen. All steps of the screen included normalization with TCF-independent Renilla luciferase controls. Dual luciferase activities were given as ratios of Firefly over Renilla values. Molecules scoring below the levels afforded by dnTCF were considered toxic.

Dual luciferase reporter assays in 293T revealed that compounds of the invention CAP1, CAP2 and CAP3 had similar repressive activities whereas comparative compound CAP4 was inactive as compared with DMSO-only controls **(****Figure 2****).**

CAP1 was evaluated for an inhibitory effect on cell proliferation, as measured by BrdU incorporation. CAP1 reduced BrdU incorporation by > 50% at 10 µM after 16h.

Compounds have been tested for the transcriptional modulation of a restricted TCF signature in human colon cancer Ls174T cells. These included the direct targets *AXIN2* (gene encoding a protein axis inhibition protein 2), *LGR5* (gene encoding encodes leucine-rich repeat-containing G-protein coupled receptor 5) and *cMYC* (a regulator gene encoding a transcription factor), which should be repressed by WNT-TCF response antagonists and *p21* (or *CDKN1A*, gene encoding a cyclin-dependent kinase inhibitor that inhibits the complexes of CDK2 and CDK1), which should be induced.

Gene expression analysis of the effects of treatment of Ls174T colon cancer cells for 16h with compounds at 10 µM concentration reveled that CAP1, CAP2 and CAP3 have inhibitory effect on WNT-TCF **(Table 2)** whereas CAP4 did not have this effect **(Table 3).**

**Table 2**

| | **Reference** | **Compound** | | |
|---|---|---|---|---|
| | **dnTCF4** | **CAP1** | **CAP2** | **CAP3** |
| ***AXIN2*** | 0.7 | 0.7 | 0.6 | 0.7 |
| ***cMYC*** | 0.4 | 0.1 | 0.6 | 0.2 |
| ***CDKN1A*** | 21.1 | 2.5 | 3.7 | 6.3 |
| ***LGR5*** | 0.2 | 0.2 | 0.7 | 0.2 |

**Table 3**

| | **Reference** | **Comparative Compound** |
|---|---|---|
| | **dnTCF4** | **CAP4** |
| ***AXIN2*** | 0.7 | 0.8 |
| ***cMYC*** | 0.4 | 0.7 |
| ***CDKN1A*** | 21.1 | 1.1 |
| ***LGR5*** | 0.2 | 0.9 |

Other withanolides or cardiac glycosides compounds have been tested as comparative compounds for their ability of modulating of an extended WNT-TCF regulated gene signature which was compared to the modulation of target gene expression induced by dnTCF4 **(Table 4),** including the following genes: *AXIN2, LGR5, cMYC, CDKN1A* Cardiac glycosides used as comparative compounds comprised: ouabain, gitoxin, gitoxigenin, digitoxin, digoxin, cymarin, oleandrin, convallatoxin and neriifolin and withanolides used as comparative compounds comprised: withanolide A, withanolide B **(****Figure 3****).** Measurements of gene expression by rt-qPCR in Ls174T colon cancer cells treated with 0.5 µM for 16h of the compounds were performed.

Measurements of gene expression in Ls174T treated with digoxin, digitoxin, ouabain, cymarin, convallatoxin, gitoxin, gitoxigenin, oleandrin or neriifolin for 16h resulted in changes in WNT-TCF target expression over DMSO-treated controls. Digoxin and the clinically approved cardiac glycoside digitoxin repressed *AXIN2,* whereas those did not repress *LGR5* and on the contrary induced *LGR5* overexpression. The lack of repressing on *LGR5* for digitoxin was confirmed even at lower compound doses (100 and 10 nM), indicating that this molecule does not harbor the same activity as CAP2. Gitoxin repressed *AXIN2,* but did not induce CDKN1 overexpression. Cymarin was ineffective and the four other comparative compounds (convallatoxin, withanolides A and B and ouabain) actually enhanced or did not influence *LGR5* expression. *cMYC* was not repressed by digoxin and *CDKN1A* was enhanced by digoxin, digitoxin, ouabain. Conversely, convallatoxin has only partial agonist activity as it enhanced *AXIN2, LGR5* and repressed *CDKN1A*, although it also repressed *cMYC.* Neriifolin enhanced both *AXIN2* and *LGR5* expression. Whereas these effects were not correlated with the extent of cell death induced after acute treatment, none of these comparative compounds induced the specific and coordinated modulation of WNT-TCF targets we observed for dnTCF or compounds of the invention and therefore were not able to mimic the activity of compounds of the invention. Further, withanolides A and B did not repress *AXIN2* and *LGR5.* Withanolide A repressed *cMYC,* but failed to enhance *CDKN1A*. On the contrary, withanolide B enhanced *CDKN1A*, but failed to repress *cMYC.* Finally, oleandrin slightly repressed *LGR5* but enhanced *AXIN2* **(Table 4).** The modulation of selected WNT-TCF, cytokine and BMP signature markers in human Ls174T colon cancer cells by withanolide A and withanolide B was also tested but none reproduced the signature changes of CAP2, arguing for specificity of compounds of the invention.

**Table 4**

| **Compound/Concentration** | ***AXIN2*** | ***LGR5*** | ***cMYC*** | ***CDKN1A*** |
|---|---|---|---|---|
| Withanolide A [0.5µM] | 0.8 | 0.8 | 0.4 | 0.7 |
| Withanolide A [5µM] | 1.8 | 1.3 | | |
| Withanolide B [0.5µM] | 1.1 | 1.3 | 1.3 | 1.9 |
| Withanolide B [5µM] | 1.3 | 1.4 | | |
| Ouabain [0.5µM] | 0.8 | 3.3 | 0.5 | 10 |
| Ouabain [5µM] | 1.0 | 1.4 | | |
| Gitoxigenin [0.5µM] | 1.0 | 0.6 | 0.4 | 0.3 |
| Digitoxin [0.5µM] | 0.6 | 2.1 | 0.2 | 1.7 |
| Digoxin [0.5µM] | 0.5 | 6.6 | 1.1 | 3.0 |
| Cymarin [0.5µM] | 1.0 | 0.9 | 1.0 | 0.9 |
| Convallatoxin [0.5µM] | 1.7 | 1.6 | 0.2 | 0.3 |
| Oleandrin [0.5µM] | 1.6 | 0.5 | 0.1 | 1.1 |
| Gitoxin [0.5µM] | 0.4 | ?? | 0.8 | 1.3 |
| Neriifolin [0.5µM] | 1.2 | 1.8 | - | - |

These results indicate that comparative withanolides or cardiac glycosides are not effective WNT-TCF inhibitors compared to compounds of the invention.

### Example 2: Reduction of cancer cells proliferation

Compounds of the invention have been tested in cancer cells cultures and their effect (tested at various concentrations) on cancer cells proliferation has been assessed by comparison of cell's growth curves.

### Cells and cultures conditions

Primary colon cells CC14 were cultured in DMEM-F12 with 10% FBS under standard *in vitro* conditions.

### Live imaging cell proliferation

Live imaging and quantification of total cell mass was performed using a Widefield plate reader, ImageXpress XL with an inbuilt temperature and CO₂ controller, and a CoolSnap HQ camera (Photometrics).

*Real-time PCR analysis* as in Example 1.

Comparison of growth curves of primary human colon cancer CC14 cells treated with CAP1, CAP2, CAP3 or DMSO alone (control) at different concentrations revealed that at 2.5 µM all three compounds have a similar negative effects on cell numbers **(****Figure 4A**). However, at 150 nM only CAP2 showed a growth repressive effect **(****Figure 4B****).** At 100 nM CAP2, but not CAP1, repressed the three canonical WNT-TCF targets in colon cancer: *AXIN2, LGR5* and *cMYC* **(Table 5).**

**Table 5**

| | **Compound [100 nM]** | |
|---|---|---|
| | **CAP1** | **CAP2** |
| ***AXIN2*** | 1.1 | 0.7 |
| ***LGR5*** | 0.8 | 0.3 |
| ***cMYC*** | 1.2 | 0.2 |

Further comparison of the effects of CAP1, CAP2 and CAP3 on CC14 cells showed higher potency of CAP2 vs. CAP1 and CAP3. The typical epithelial island morphology of these cells was maintained after 24h treatment with 1 µM CAP1 or CAP3 but totally disrupted after treatment with 1 µM CAP2 for the same time. Similar phenotypes were only observed at 10 µM of CAP1 or 5 µM of CAP3. CAP2 was thus chosen for further detailed analyses.

These results indicate that compounds of the invention can effectively prevent cancer cells proliferation, with CAP2 being the most potent at lower concentrations.

### Example 3: Inhibition of cancer stem cell clonogenicity and induction of apoptosis

Compounds of the invention have been tested in cancer cells cultures and their effect on regulation of direct TCF targets and number of stem cell-derived spheroids was assessed. Further, the ability of the compounds to induce apoptosis in cancer cells cultures was analyzed by assessing activated Caspase3 labeling.

### Cells and cultures conditions

Colon cancer cell lines DLD1 and LS174T cultured in DMEM-F12 with 10% FBS under *standard in nitro* conditions.

### Clonal spheroid assays

Cells were treated with different concentrations of compounds for 16h in 2D adherent culture, washed, harvested and plated in suspension with serum-free DMEM-F12 media with B27 supplement (1:50 dilution) at 1 cell/well in 96-well format in triplicates. Plated cells were monitored to ensure the presence of single cells in each well and spheroids counted and photographed after 10 days for each round.

### Live imaging for total cell mass and activated Caspase assays

Live imaging was performed as in Example 3. Cell were plated with different concentrations of compounds in a 96-well format and incubated in standard conditions for 6h and imaged. Active (cleaved) Caspase-3⁺ apoptosis was determined using the BD Pharmigen kit and FACS (fluorescence-activated cell sorting).

*Microarrays* and *Real-time PCR analysis* as in Example 1

In order to confirm that the inhibitory effects of CAP2 on the WNT-TCF pathway confers the ability to block colon cancer stem cell self-renewal, which is WNT-TCF *dependent in vitro* (Vermeuen et al., 2010, Nat Cell Biol., 12(5): 468-76*;* Krausova et al., 2014, Cell Signal., 26(3): 570-9*),* Ls174T as well as DLD1 cells were treated in 2D culture with different doses of CAP2 and subsequently the cells were plated as suspension 3D clonal cultures after washing off the compound. Cells treated in 2D showed the expected regulation *of AXIN2,* a direct TCF target (Lsl74T treated with CAP2 at 2.5µm shoved 0.4 expression level as compared to control levels of *AXIN2;* DLD1 treated with CAP2 at 2.5µm shoved 0.2 expression level as compared to control *AXIN2),* and the resulting number of stem cell-derived spheroids was decreased with an IC₅₀ of <1.25µM **(****Figure 5****).** Analyses of activated Caspase3 labeling as a marker of apoptosis revealed an increase number of labeled cells by FACS starting at 2.5µM of CAP2 as compared with DMSO-treated Ls174T and DLD1 cells **(****Figure 6****).** It is worth noting that level at which it is possible to first detect a significant increase in apoptosis is at 2.5µM CAP2, which is higher than that which changes cell proliferation assessed by BrdU incorporation (1.25 µM CAP2) and gene expression changes (see below), indicating that the observed effects with CAP2 at low doses are unlikely due to generic cytotoxicity.

These results indicate that CAP2 can block colon cancer stem cell clonogenicity and induce apoptosis.

### Example 4: Repression of WNT-TCF targets in multiple human cancer cells

Compounds of the invention have been tested at multiple concentrations, in various cancer cells cultures by testing their effect on TCF target gene expression.

### Cells and cultures conditions

Primary colon cells CC14, CC36 and mCC11, colon cancer cell lines DLD1, HT29, Ls174T, melanoma cell line MeWo, glioblastoma cell lines U87 and U251, and the lung cancer cell line H358 were cultured in DMEM-F12 with 10% FBS under standard *in nitro* conditions.

### Microarrays and Real-time PCR analysis as in Example 1

The antagonistic action of CAP2 on the WNT-TCF pathway was first tested at the level of TCF target gene expression changes in multiple human colon cancer cells. These included three well characterized cells lines, LS174T, DLD1 and HT29, harboring mutations in β-CATENIN (Ls174T) or in APC (DLD1, HT29), as well as in three not cloned primary colon cancer cells, two from bowel tumors (CC14 and CC36) and one from a liver metastasis (mCCll). Whereas there were small context-differences, the canonical colon cancer WNT-TCF targets *AXIN2* and *LGR5* were generally repressed at 2.5-5 µM, with strong repression of *LGR5* at 1.25 µM in Ls174T and CC36 **(Table 6).** Beyond colon cancer, CAP2 treatment repressed the levels *of AXIN2* and *LGR5* in human U87 and U251 glioblastoma cells, of *AXIN2* and *LEF1. LEF1* was used as an additional WNT-TCF target gene in cell lines where *LGR5* is not expressed or not regulated by WNT-TCF signaling such as for example melanoma (since LGR5 was not detected, *LEF1* gene encodes lymphoid enhancer-binding factor 1) in MeWo human melanoma cells, and in H358 human lung cancer cells **(Table 6).** Repression of *LGR5* in glioblastoma, *of AXIN2* and *LEF1* in melanoma, and *of LEF1* in lung cancer was already observed at 1.25 µM after 16h **(Table 6).** All values are normalized ratios with DMOS-controls, which were equated to 1. All analyses shown in this Table 6 were performed with the third CAP2 batch.

**Table 6**

| **CAP2 Concentration** | **Cell Type** | ***AXIN2*** | ***LGR5*** | ***LEF1*** |
|---|---|---|---|---|
| Colon cancer | | | | |
| [1.25µM] | Ls174T | 0.6 | 0.2 | - |
| [2.5µM] | Ls174T | 0.4 | 0.1 | - |
| [5µM] | Ls174T | 0.2 | 0.03 | - |
| [10µM] | Ls174T | 0.1 | 0.1 | - |
| [1.25µM] | DLD1 | 1.2 | 1.6 | - |
| [2.5µM] | DLD1 | 0.2 | 0.3 | - |
| [5µM] | DLD1 | 0.3 | 0.1 | - |
| [10µM] | DLD1 | 0.1 | 0.04 | - |
| [1.25µM]; | HT29 | 0.8 | 0.8 | - |
| [2.5µM] | HT29 | 0.9 | 0.7 | - |
| [5µM] | HT29 | 0.8 | 0.3 | - |
| [10µM] | HT29 | 0.4 | 0.1 | - |
| [1.25µM] | CC14 | 0.9 | 1.0 | - |
| [2.5µM] | CC14 | 1.5 | 0.8 | - |
| [5µM] | CC14 | 0.7 | 0.1 | - |
| [10µM] | CC14 | 0.7 | 0.03 | - |
| [1.25µM] | CC36 | 1.2 | n.d. | - |
| [2.5µM] | CC36 | 0.6 | n.d. | - |
| [5µM] | CC36 | 0.4 | n.d. | - |
| [10µM] | CC36 | 0.4 | n.d. | - |
| [1.25µM] | mCC11 | 0.6 | 0.8 | - |
| [2.5µM] | mCC11 | 0.5 | 0.3 | - |
| [5µM] | mCC11 | 0.3 | 0.1 | - |
| [10µM] | mCC11 | 0.3 | 0.04 | - |

| Glioblastoma | | | | |
|---|---|---|---|---|
| [1.25µM] | U87 | 0.7 | 0.3 | - |
| [2.5µM] | U87 | 0.5 | 0.3 | - |
| [5µM] | U87 | 0.5 | 0.2 | - |
| [10µM] | U87 | 0.6 | 0.2 | - |
| [1.25µM] | U251 | 0.9 | 0.5 | - |
| [2.5µM] | U251 | 0.8 | 0.3 | - |
| [5µM] | U251 | 0.7 | 0.2 | - |
| [10µM] | U251 | 0.5 | 0.2 | - |

| Melanoma | | | | |
|---|---|---|---|---|
| [1.25µM] | MeWo | 0.4 | n.d. | 0.1 |
| [2.5µM] | MeWo | 0.6 | n.d. | 0.2 |
| [5µM] | MeWo | 0.3 | n.d. | 0.1 |
| [10µM] | MeWo | 0.6 | n.d. | 0.1 |

| Lung cancer | | | | |
|---|---|---|---|---|
| [1.25µM] | H358 | 0.8 | n.d. | 0.4 |
| [2.5µM] | H358 | 0.7 | n.d. | 0.6 |
| [5µM] | H358 | 0.6 | n.d. | 0.4 |
| [10µM] | H358 | 0.4 | n.d. | 0.2 |

| | | | | |
|---|---|---|---|---|
| n.d.- non detectable | | | | |

Ls174T, DLD1 and mCC11 cells were treated with different concentrations of CAP2 and the effects on further TCF targets were tested **(Table 7).** The direct target *LGR5* were very sensitive to CAP2 repression. As with dnTCF4 *(*van de Wettering et al., 2002, Cell., 111(2): 241-50*;* Varnat et al., 2010, EMBO Mol Med, 2(11): 440-57*), CDKN1A* was enhanced at all doses tested **(Table 7).** All values are normalized ratios with DMOS-controls, which were equated to 1.

**Table 7**

| **CAP2 - Concentration/Cells** | ***cMYC*** | ***LGR5*** | ***AXIN2*** | ***ASCL2*** | ***LEF1*** | ***CDKN1A*** |
|---|---|---|---|---|---|---|
| [1.25µM]; Ls174T | 0.04 | 0.3 | 0.7 | 1.1 | 1.2 | 3.5 |
| [2.SµM]; Ls174T | 0.1 | 0.2 | 0.4 | 0.4 | 0.7 | 5.4 |
| [5µM]; Ls174T | 0.1 | 0.1 | 0.3 | 0.1 | 0.5 | 11.4 |
| [10µM]; Ls174T | 0.01 | 0.1 | 0.1 | 0.1 | 0.3 | 7.6 |
| [1.25µM]; DLD1 | 0.5 | 0.5 | 0.8 | 0.2 | 0.4 | 7.2 |
| [2.5µM]; DLD1 | 0.2 | 0.3 | 0.6 | 0.2 | 0.3 | 8.6 |
| [5µM]; DLD1 | 0.001 | 0.1 | 0.5 | 0.3 | 0.1 | 9.0 |
| [10µM]; DLD1 | 0.0002 | 0.1 | 0.2 | 0.3 | 0.1 | 6.1 |
| [1.25µM]; mCC11 | 0.5 | 0.8 | 0.6 | 0.3 | 0.3 | 1.4 |
| [2.5µM]; mCC11 | 0.3 | 0.3 | 0.5 | 0.2 | 0.4 | 2.7 |
| [5µM]; mCC11 | 0.1 | 0.1 | 0.3 | 0.1 | 0.5 | 6.5 |
| [10µM]; mCC11 | 0.1 | 0.04 | 0.3 | 0.02 | 0.6 | 5.3 |

Together, these results indicate that CAP2 treatment represses WNT-TCF targets in a context-specific and a concentration-dependent manner in multiple human cancer cells (colon cancer, glioblastoma, melanoma, lung cancer).

### Example 5: Specific repression of WNT-TCF targets by the compounds of the invention

The specificity of the compounds of the invention on WNT-TCF targets was tested in so-called rescue test of compound-induced WNT-TCF targets repression as described below.

### Epistatic rescue experiments

Lentivectors expressing a TCF4^{VP16} protein in a vector independently expressing GFP, or vector alone, plus reporter plasmids were transfected into cells. 72h after transfection cells were visually checked for expression of GFP. Independent batches of cells with >80% GFP expression were chosen for further experimentation and treated with or without compounds in DMEM-F12 (with 2.5% FBS) for 16h and collected in Trizol.

*Cells and cultures conditions* as in Example 4.

*Microarrays* and *Real-time PCR analysis* as in Example 1

If the effects of CAP2 on WNT-TCF targets were specific, an activated form of TCF should rescue the repression since CAP2 is likely to act downstream of the APC destruction complex since it was found using an N'Δ form of βCATENIN that is APC insensitive. To activate this last step of canonical WNT signaling, a fusion between TCF and the potent transactivation domain of the viral VP16 protein, TCF^{VP16} *(Melotti et al., 2014, supra*), was used to directly and positively regulate TCF targets. Analyses in three colon cancer cell types revealed the complete epistatic rescue of the expression *of AXIN2* from CAP2 (at 5µM concentration) repression in cells expressing TCF^{VP16} **(Table 8).** Rescue of *LGR5, cMYC* and *ASCL2* repression by CAP2 (at 5µM) was also observed although there was variability between the cell types, similarly to the repression of *CDKN1A* **(Table 8),** although in all cases it reversed its enhancement by CAP2 treatment **(Table 8).**

**Table 8**

| | **TCF^{VP16}+ CAP2 / control + CAP2** | | |
|---|---|---|---|
| | **Ls174T** | **DLD1** | **mCC11** |
| ***AXIN2*** | 16.1 | 3.4 | 21.9 |
| ***LGR5*** | 0.7 | 1.4 | 2.9 |
| ***cMYC*** | 2.7 | 0.8 | 3.3 |
| ***ASCL2*** | 1.0 | 1.5 | 3.5 |
| ***CDKN1A*** | 0.5 | 0.4 | 0.8 |

Together, these results indicate that CAP2 treatment represses WNT-TCF targets downstream of the APC/βCATENIN complex.

### Example 6: Compounds of the invention induce genome-wide transcriptomic changes in cancer cells

The effects of compounds of the invention on cancer cells' transcriptome were assessed by evaluating extended WNT-TCF target gene modulation.

*Cells and cultures conditions as in Example 4.*

### Microarrays and Real-time PCR analysis as in Example 1

To test the effects of CAP2 on the transcriptome in a genome-wide manner microarray analyses with Ls174T cells were performed as these have been previously used to determine the genome-wide changes driven by dnTCF activity *(*van der Flier et al., 2007, Gastroenterology, 132(2): 628-32), the genetic benchmark use here. Ls174T cells were treated with 5 µM CAP2 (batch 3) for 16h, and their transcriptome compared with that of controls treated with DMSO under the same conditions. This dose was chosen since it results in extended WNT-TCF target gene modulation **(Table 6).**

Global analyses in CAP2-treated Ls174T cells in triplicates using a 2-fold threshold over control showed large cohorts of down-regulated and up-regulated probes over 2-fold and with p<0.05. This included the downregulation of a number of archetypal and direct TCF targets, including *LGR5* (15-fold), *ASCL2* (9-fold) and *AXIN2* (4-fold) **(Table 9),** also confirming the differential regulation of WNT-TCF targets observed above **(Table 7).**

**Table 9**

| **Gene name** | **Fold change** | **P value** |
|---|---|---|
| ***AXIN2*** | -4 | 7E-08 |
| ***ASCL2*** | -9 | 1E-05 |
| ***LGR5*** | -15 | 2E-07 |
| ***cMYC*** | -10 | 7E-10 |
| ***CDKN1A*** | 9 | 1E-09 |

To more directly analyze the effects of CAP2 on TCF targets, the previously determined 106-gene set of TCF targets in Ls174T cells was used *(van der Flier et al., 2007, supra*) that were detectable in previously presented microarrays. Mining current microarray data for probes corresponding to these genes, which were repressed by 1.5-fold or more, showed that CAP2 treatment repressed 70% of the gene set **(****Figure 7****).** CAP2 repressed a central set of 36 genes that included the canonical WNT-TCF targets *AXIN2, LGR5, ASCL2*and *cMYC.*

Given that CAP2 blocks WNT-TCF responses in multiple cancer cell types (Example 4), the behavior of a set of 15 core WNT-TCF targets that are repressed by dnTCF1/4 in both Ls174T (with a βCATENIN activating mutation) and DLD1 (with a APC loss of function mutation) cells according to *van der Flier et al., 2007, supra*) was tested. Microarray data (using a cutoff of 75 detection units) of 15-gene core TCF target set was shown to be repressed by CAP2 **(Table 10).** Values for dnTCF1/4 according to Table 1 of *van der Flier et al., 2007, supra).*

**Table 10**

| | **Fold change, Ls174T cells** | |
|---|---|---|
| **Gene name** | **CAP2** | **dnTCF1/4** |
| Microarray | | |
| ***AXIN2*** | -4.1 | -4.9 |
| ***LGR5*** | -15.1 | -9.8 |
| ***ASCL2*** | -9.1 | -24 |
| ***cMYC*** | -10.2 | -3.2 |

Together, these data indicate that CAP2 treatment induces genome-wide transcriptomic changes in human colon cancer cells that include the canonical WNT-TCF targets *AXIN2, LGR5, ASCL2* and *cMYC.*

### Example 7: CYCLIN D1 levels changes in cancer cells treated with the compound of the invention

To test the mode of action of compounds of the invention on WNT-TCF signaling, the levels of direct TCF target CYCLIN D1 in cancer cells treated with the compounds were measured.

*Cells and cultures conditions as in Example 4.*

*Western blot* performed according to standard methods.

The effect of CAP2 on WNT-TCF signaling the levels of direct TCF target CYCLIN D1 were measured since it is useful to measure TCF response (*Melotti et al., 2014, sura*). Treatment of DLD1 cells with 5 µM CAP2 (batch 3) resulted in a decrease in the level of TCF target CYCLIN D1 and this repression was reversed by treatment with 15 nM okadaic acid (OA), which blocks the activity of the serine protein phosphatases PP2A and PP1 (**Figure 8****).**

These data suggest that CAP2 represses the TCF target through a mechanism involving active protein phosphatases, like PP2A and/or PP1 to exert its repressive effect on the WNT-TCF pathway and may thus reduce active phospho-βCATENIN/TCF complexes.

### Example 8: DLD1 colon cancer xenograft model

The ability of the compounds of the invention to inhibit tumor growth was tested in mice xenografted with the TCF-dependent cancer cells.

### Xenograft assays in Nude mice

0.5x10⁶ DLD1/ GFP⁺ (cells expressing green fluorescent protein) cells were injected *per* flanks into 6 week-old female Nude mice (Janvier Labs). CAP2 (10mg/kg) complexed with cyclodextrine as vehicle, or vehicle alone, were injected daily intra-peritoneally (IP) once the tumors were palpable (25-50 mm³). Tumor volumes were measured every other day with a caliper. Animals were sacrificed at the end of experiment and tumors harvested.

### Drug preparation for in vivo experiment

CAP2 from four independent batches supplied by Analyticon Discovery was dissolved in DMSO and suspended in 45% beta-cyclodextrin. 45% beta-cyclodextrin was used as vehicle for controls. 150µl of drug or vehicle alone was administered per mouse daily.

*Microarrays* and *Real-time PCR analysis* as in Example 1

The ability of CAP2 to inhibit tumor growth was tested in NUDE mice xenografted with the TCF-dependent colon cancer cell line DLD1 *(Varnat et al., 2010, supra).* This cell line was chosen as it is dnTCF4-responsive *in vivo:* DLD1 xenograft growth is inhibited by dnTCF4 (*Varnat et al., 2010, supra*).

GFP⁺ DLD1-engrafted mice were treated with cyclodextrin-conjugated CAP2 at 10mg/kg *via* IP every second day starting at the time when tumors were palpable. Systemic delivery of CAP2, but not cyclodextrin carrier alone, resulted in the blockade of tumor growth without any visible side effects in two independent experiments **(****Figure 9** **A, B).** Analyses of gene expression using human-specific primers in treated tumors (using the excised area where remnant DLD-GFP⁺ cells could be found) confirmed the repression of human *AXIN2, ASCL2* and *LGR5* **(Table 11**).

**Table 11**

| | ***in vitro*** | |
|---|---|---|
| | Ls174T | DLD1 |
| ***AXIN2*** | 0.3 | 0.5 |
| ***ASCL2*** | 0.1 | 0.3 |
| ***LGR5*** | 0.1 | 0.1 |

### Example 9: Induction of the long-lasting repression of cancer stem cells and WNT-TCF targets

The ability of the compounds of the invention to induce the long-lasting repression of cancer stem cells and WNT-TCF targets was assessed in cultures of tumor-derived cells.

### Xenograft assays in Nude mice as in Example 8

### Drug preparation for in vivo experiment as in Example 9

### Microarrays and Real-time PCR analysis as in Example 1

### Clonal spheroid assays as in Example 3

Four DLD1 regressed tumors were monitored after cessation of CAP2 treatment to determine recurrence after drug withdrawal. After 90 days following drug removal only one CAP2-repressed tumor re-grew, suggesting a penetrant effect of CAP2 inhibition. Tumor regrowth, however, could be due in principle to insufficient drug dosage or to the development of drug resistance. To test these possibilities the recurrent tumor was thus excised and the cells plated *in vitro* to test for the possible development of CAP2 resistance. One control *in vivo* cyclodextrin-treated tumor taken before reaching the legal (at around 21 day) limit was similarly processed to serve as control. *In vitro*, treatment of tumor-derived cells with CAP2 resulted in a concentration-dependent repression of TCF targets below the levels of DMSO-treated control cells in each case **(Table 12,** **Figure 10****),** indicating the absence of drug resistance. The levels of WNT-TCF targets were generally lower in cells derived from tumors that were exposed *in vivo* to CAP2 in comparison to those in cells from tumors that were not treated with CAP2 **(****Figure 10****).** *In vitro* treatment with DMSO of cells derived from *in vivo* CAP2-treated tumors resulted in TCF target levels that were lower than those observed in *in vitro* DMSO-treated cells from *in-vivo*-cyclodextrin-treated tumors **(****Figure 9****).** This consistent difference in the levels of several WNT-TCF targets raised the possibility that whereas tumor recurrence might be due to incomplete initial dosage, the recurring tumor harbored the memory of previous CAP2 treatment.

**Table 12**

| **DLD1 control tumor dissociated treated *in vitro*** | ***AXIN2*** | ***LGR5*** | ***ASCL2*** | ***cMYC*** | ***LEF1*** |
|---|---|---|---|---|---|
| CAP2 [2.5µM] | 0.6 | 0.7 | 0.1 | 0.3 | 0.4 |
| CAP2 [5µM] | 0.6 | 0.4 | 0.1 | 0.3 | 0.3 |

| **DLD1 CAP2-treated tumor dissociated re-treated *in vitro*** | | | | | |
|---|---|---|---|---|---|
| CAP2 [2.5µM] | 0.4 | 0.6 | 0.05 | 0.2 | 0.4 |
| CAP2 [5µM] | 0.3 | 0.3 | 0.1 | 0.1 | 0.3 |

To directly test for a long-lasting repression of WNT-TCF targets by CAP2 clonogenic properties of cancer stem cells were tested. DLD1 and LS174T cells were first treated with CAP2 in 2D culture *in vitro* followed by removal of the drug, washing with PBS, and plating under non-adherent 3D conditions for clonal spheroid growth in the absence of the drug. After primary spheroid growth, the spheroids were dissociated and replated as single cells for secondary and subsequent clonogenic spheroid formation, also in the absence of the compound of the invention. As negative control DMSO-treatment was used.

Quantification revealed a dose-dependent decrease in primary spheroids after treatment with CAP2, normalized over DMSO controls **(****Figure 11A****).** CAP2 treatment of 2D cells cultures resulted in a decrease in primary 3D clonogenic spheroids thus indicating for a decrease in number clonogenic stem cells. The frequency of spheroids derived from CAP2 treated cells continued to show reduced frequencies in the long-term absence of drug as tertiary clonogenic spheroids of Ls174T secondary spheroids also showed diminished frequencies **(****Figure 11B****).** Secondary and tertiary clonal spheroid assays revealed that the repression imposed by CAP2 is long-lasting. CAP2 pretreatment of Ls174T cells at 5µM leads to the exhaustion of clonogenic cancer stem cells after three passes **(****Figure 11B****).** Treatment in 2D culture resulted in the expected concentration-dependent repression of WNT-TCF targets by CAP2 **(Table 13).** Further, analyses of their expression in secondary spheroids revealed sustained repression in CAP2- pretreated samples, as compared with DMSO controls. These findings suggest the association of repressed clonogenic growth with repressed WNT-TCF signaling responses **(Table 13).**

**Table 13**

| | ***AXIN2*** | ***cMYC*** |
|---|---|---|
| **CAP2 [2.5µM] DLD1 2D** | 0.2 | 0.1 |
| **CAP2 [2.5µM] DLD1 secondary 3D spheroids** | 0.5 | 0.4 |

Together these data suggest a novel WNT-TCF response blocking activity of CAP2 with the unique characteristic to repress signaling responses well beyond the time of drug delivery, possibly by imposing a stable epigenetic change.

### Example 10: Modulation of a set of chromatin components and remodelers

The ability of the compounds of the invention to modulate the expression of chromatin components and remodelers was assessed by measuring revealed a cohort of genes in cancer cells treated with the compounds.

### HISTONE 3 assays

EpiQuick™ HISTONE 3 modification colorimetric assays (Epigentek) were used to quantify the levels of overall HISTONE 3 modifications in Ls174T cells treated with same drug dose used for genomic analyses (5µM CAP2 in DMSO, or DMSO only as control for 16h). Results were read on a Victor screening robot and levels normalized over total H3 levels as indicated by the manufacturer.

Mining the transcriptome of CAP2-treated cells for CAP2-induced expression changes the public Reactome database for a chromatin organization cluster expanded to include related family members (94 probes) was used. This analysis (using only specific _a and _a_at probes) revealed a cohort of 76 genes with CAP2-specific expression level changes by 3-fold or more **(****Figure 12****).** Of these, *HISTONE* 2 and 3A genes were up-regulated 5-fold or more. Conversely *ZMYND8, EED, SMARCAL1, TBL1XR1, TAF5Ls,* were down-regulated 5-fold or more. These genes are of interest as they are coding for: the bromodomain factor *ZMYND8* (repressed 28-fold) involved in DNA repair (Gong et al., 2015, Genes Dev., 29(2): 197-211) and implicated in cancer as a fusion protein (Panagopoulos et al., 2013, PLoS One, 8(5): e63663*),* the key Polycomb repressive complex 2 (PRC2) component *EED* (repressed 8-fold), which associates with EZH2 (which trimethylates H3K27 causing gene silencing) and also functions in the coordination of PRC complexes (Cao et al., 2014, Nat Commun, 5: 3127), the SWI/SNF chromatin remodeling factor *SMARCAL1* (repressed 6-fold) involved in genome integrity (Bansbach et al., 2009, Genes Dev., 15;23(20): 2405-14); and other important multiple HISTONE genes (upregulated 7-to 52-fold).

Together, and given the key roles of some of these genes in modulating chromatin and gene expression, the data raised the possibility that CAP2 alters the epigenetic landscape of cancer cells, including cancer stem cells, and imposes long- lasting WNT-TCF pathway repression. Following the changes observed in the levels of expression of epigenetic components and modulators by CAP2 treatment (Example 6), the effect of CAP2 on specific HISTONE marks was tested by analyzing multiple HISTONE H3 modifications with the Epiquik™ kit in DLD1 cells treated with CAP2 (5µg of batch 3), as compared to control DMSO-treated cells. Whereas most modifications showed little variation, after normalization to total HISTONE H3 levels, after treatment with DMSO, mono-methylated lysine 4 (H3K4Mel) was significantly (p<0.05) reduced by 40% on average by CAP2 treatment. Since H3K4Mel has been also associated to hypomethylated DNA regions during aging *(*Fernandez et al., 2015, Genome Res., 25:27-40*),* it is possible that CAP2 treatment may lead to the repression of gene expression by hypermethylation and loss of enhancer activity.

These data indicate that CAP2 regulates a large number of chromatin remodelers and may impose a long-lasting repression of WNT-TCF targets and clonogenic cancer stem cell growth.

### Example 11: Effects on WNT-TCF signaling and epigenetic regulators

The molecular pathways modulated by CAP2 action were assessed by analysis of gene expression encoding WNT-TCF targets, BMP/TGFβ signaling, chemokines/cytokines and epigenetic regulators at two time points and comparing them to action of dnTCF on the same genes.

### Microarrays and Real-time PCR analysis as in Example 1

Nine components of the three pathways (WNT-TCF, BMP/TGFβ and chemokine/cytokine), and the top 6 repressed epigenetic regulators (*ZMYND8, EED, SMARCAL1, TBLIXR1, SETD6* and *TAF5L)* **(****Figure 12****)** were tested for their modulation by dnTCF and their timed response to CAP2 treatment. Analyses of the steady-state effects of direct TCF repression by dnTCF in DLD1 cells, 7-days post-treatment, used for *in vivo* experiments, revealed the repression of *AXIN2* and *LGR5,* confirming repression of WNT-TCF responses **(Table 14).** DnTCF also repressed the expression of *BMP4, SMAD6, IL8, CXCL3* and *CXCL5,* but expression of *IL33* was enhanced. DnTCF did not repress *ZMYND8*, *EED, SMARCAL1, TBLIXR1, TAF5L* or *SETD6* and instead it enhanced the expression of all but that of *SMARCAL1.* Analysis of selected genes related to WNT-TCF, BMP/TGFβ signaling, cytokine/chemokine and epigenetic regulator genes after 2 or 4h following CAP2 treatment (CAP2-batch 3, at 5µM in Ls174T cells used for the transcriptomic analyses) revealed that *AXIN2* and *LGR5,* used here as controls for canonical WNT pathway regulation, were already repressed at these time points. *BMP4* and *ID2* were also repressed at 2 and 4h but *SMAD6* was repressed only at 4h. *CXCL3* was already repressed at 2h, *IL8* was little affected and *IL33* and *CXCL5* were enhanced at 2h. At 4 h only *CXCL3* was repressed and the other three cytokine/chemokine genes tested (*IL8, IL33* and *CXCL5*) were neither enhanced nor repressed. None of the 6 epigenetic regulators tested were repressed at 2h, instead all but *TAF5L* were transiently enhanced at 2h as with dnTCF and all but *SMARCAL1* were subsequently repressed at 4h **(Table 14).** All values are normalized ratios with DMSO-controls, which were equated to 1

**Table 14**

| | **dnTCF4/c [explain /c]** | **CAP2 - batch 3 [5µM]** | |
|---|---|---|---|
| | 7 days post-infection | 2h post-treatment | 4h post-treatment |
| | WNT-TCF targets | | |
| ***AXIN2*** | 0.3 | 0.2 | 0.1 |
| ***LGR5*** | 0.3 | 0.7 | 0.1 |

| | BMP/TGFβ | | |
|---|---|---|---|
| ***BMP4*** | 0.4 | 0.4 | 0.1 |
| ***ID2*** | 1.2 | 0.7 | 0.1 |
| ***SMAD6*** | 0.5 | 1.3 | 0.4 |

| | Chemokine/cytokine | | |
|---|---|---|---|
| ***IL8*** | 0.5 | 0.7 | 0.8 |
| ***IL33*** | 3.3 | 1.4 | 1.1 |
| ***CXCL3*** | 0.6 | 0.2 | 0.1 |
| ***CXCL5*** | 0.3 | 1.3 | 1.1 |

| | Epigenetic regulators | | |
|---|---|---|---|
| ***ZMYND8*** | 1.5 | 1.3 | 0.5 |
| ***EED*** | 1.4 | 1.5 | 0.5 |
| ***SMARCAL1*** | 1.1 | 1.6 | 0.8 |
| ***TBL1XR1*** | 2.2 | 2.6 | 0.6 |
| ***SETD6*** | 1.7 | 2.0 | 0.7 |
| ***TAF5L*** | 1.6 | 0.8 | 0.3 |

Together, these results suggest that the effects of CAP2 treatment on BMP/TGFβ and cytokine/chemokine pathways are at least partially secondary to effects on WNT-TCF with varying kinetics. Moreover, CAP2 appears to have an early dnTCF-like enhancement of epigenetic regulators followed by their WNT-TCF-independent repression.

The WNT-TCF-independent repressive activity of CAP2 on epigenetic regulators may be extrapolated to other cancer cells as it was also detected in HT29 and CC14 human colon cancer cells, in MeWo human melanoma cells as well as in U87 and U251 human glioma cells. In all these different human cancer cells, CAP2 (at 0.5 µM) modulated the expression of *ZMYND8, SMARCAL1, TBLIXR1, SETD6* and *TAF5Ls* **(Table 15).**

**Table 15**

| | Colon cancer | | Melanoma | Glioblastoma | |
|---|---|---|---|---|---|
| | HT29 | CC14 | MeWo | U87 | U251 |
| ***ZMYND8*** | 0.1 | 0.1 | 0.1 | 0.1 | 0.4 |
| ***EED*** | 0.3 | 0.3 | 0.5 | 0.2 | 0.8 |
| ***SMARCAL1*** | 0.2 | 0.2 | 0.2 | 0.2 | 0.5 |
| ***TBL1XR1*** | 0.02 | 0.2 | 0.2 | 0.2 | 0.6 |
| ***SETD6*** | 0.2 | 0.4 | 1 | 0.3 | 0.9 |
| ***TAF5L*** | 0.4 | 0.5 | 0.8 | 0.5 | 1.1 |

These results indicate that CAP2 action could be mediated by WNT-TCF signaling repression that leads to secondary effects on BMP/TGFβ and cytokine/chemokine pathways and WNT-TCF-independent repression of epigenetic regulators. Therefore, CAP2 may have therapeutic potential due to its dual action as a WNT-TCF blocker and as an epidrug, leading to sustained WNT-TCF response repression.

### Sequence listing

Amino acid sequence of AXIN2 Fw
   SEQ ID NO:1: CTCCTTATCGTGTGGGCAGT
Amino acid sequence of AXIN2 Rev
   SEQ ID NO:2: CCAACTCCAGCTTCAGCTTT
Amino acid sequence of ASCL2 Fw
   SEQ ID NO:3: GCGTTCCGCCTACTCGT
Amino acid sequence of ASCL2 Rev
   SEQ ID NO:4: GGCTTCCGGGGCTGAG
Amino acid sequence of LEF1 Fw
   SEQ ID NO:5: AACCTCTCAGGAGCCCTACC
Amino acid sequence of LEF1 Rev
   SEQ ID NO:6: CACGGGCACTTTATTTGAT
Amino acid sequence of LGR5 Fw
   SEQ ID NO:7: GGAGCATTCACTGGCCTTTA
Amino acid sequence of LGR5 Rev
   SEQ ID NO:8: CTGGACGGGGATTTCTGTTA
Amino acid sequence of cMYC Fw
   SEQ ID NO:9: TGGTCTTCCCCTACCCTCT
Amino acid sequence of cMYC Rev
   SEQ ID NO:10: GATCCAGACTCTGACCTTTT
Amino acid sequence of CDKN1A/p21 Fw
   SEQ ID NO:11: GACTCTCAGGGTCGAAAACG
Amino acid sequence of CDKN1A/p21 Rev
   SEQ ID NO:12: AAGATGTAGAGCGGGCCTTT
Amino acid sequence of EPHB2 Fw
   SEQ ID NO:13: ATGCGGAAGAGGTGGATGTA
Amino acid sequence of EPHB2 Rev
   SEQ ID NO:14: CCTTGAAAGTCCCAGATGGA
Amino acid sequence of EPHB3 Fw
   SEQ ID NO:15: TGCCACTCAAGCTCTACTGC
Amino acid sequence of EPHB3 Rev
   SEQ ID NO:16: GTTATTGTGGCAGGTGCAGA
Amino acid sequence of SOX4 Fw
   SEQ ID NO:17: AAACCAACAATGCCGAGAAC
Amino acid sequence of SOX4 Rev
   SEQ ID NO:18: GTTCATGGGTCGCTTGATGT
Amino acid sequence of ZNRF3 Fw
   SEQ ID NO:19: CGGGTCATCCCCTGTACTC
Amino acid sequence of ZNRF3 Rev

   SEQ ID NO:20: ACGTGAGAGGTTGCTGGTCT
Amino acid sequence of KITLG Fw
   SEQ ID NO:21: TTCCATCATAGACAAACTTGTGA
Amino acid sequence of KITLG Rev
   SEQ ID NO:22: GTAAAGAGCCTGGGTTCTGG
Amino acid sequence of CXCL3 Fw
   SEQ ID NO:23: CGTCCGTGGTCACTGAACT
Amino acid sequence of CXCL3 Rev
   SEQ ID NO:24: ATGACTTCGGTTTGGGCG
Amino acid sequence of IL33 Fw
   SEQ ID NO:25: TGAGTCTCAACACCCCTCAA
Amino acid sequence of IL33 Rev
   SEQ ID NO:26: GTTGGCATGCAACCAGAAGT
Amino acid sequence of ID3 Fw
   SEQ ID NO:27: ACCTTCCCATCCAGACAGC
Amino acid sequence of ID3 Rev
   SEQ ID NO:28: CTTCCGGCAGGAGAGGTT
Amino acid sequence of BMP4 Fw
   SEQ ID NO:29: GATCCACAGCACTGGTCTTG
Amino acid sequence of BMP4 Rev
   SEQ ID NO:30: GGGATGCTGCTGAGGTTAAA
Amino acid sequence of ZMYND8 Fw
   SEQ ID NO:31: CGAGACCCAGAGTAAAGCCAT
Amino acid sequence of ZMYND8 Rev
   SEQ ID NO:32: GATGTATCCGCATAGTCAGGG
Amino acid sequence of EED Fw
   SEQ ID NO:33: GTGACGAGAACAGCAATCCAG
Amino acid sequence of EED Rev
   SEQ ID NO:34: TATCAGGGCGTTCAGTGTTTG
Amino acid sequence of SMARCAL1 Fw
   SEQ ID NO:35: ACAGCATCAGAGGACTAGCTC
Amino acid sequence of SMARCAL1 Rev
   SEQ ID NO:36: CACTGGCTTACAAGACTCCCT
Amino acid sequence of TBL1XR1 Fw
   SEQ ID NO:37: CCAGTTCCCATTTCCTGCCA
Amino acid sequence of TBL1XR1 Rev
   SEQ ID NO:38: ACACCACAAAAGGAGGCACTT
Amino acid sequence of SETD6 Fw
   SEQ ID NO:39: GGAGGGAACAGCAAGCCTTA
Amino acid sequence of SETD6 Rev
   SEQ ID NO:40: TCAGGGAACAGGGAAACTGC
Amino acid sequence of SETD6 Fw
   SEQ ID NO:41: CAGGCAGAACCCCAGCAATA
Amino acid sequence of SETD6 Rev
   SEQ ID NO:42: CACTGTGCTCTTCGGACTGT

## Claims

1. A compound of Formula (I): 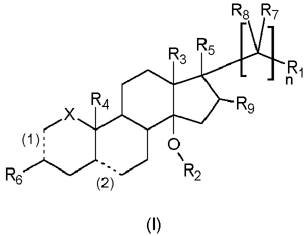 wherein X is selected from C=O, -CH or an optionally substituted C₁-C₆ alkyl; R¹ is an optionally substituted lactone; R² is selected from H and optionally substituted C₁-C₆ alkyl; R³ and R⁴ are independently selected from H, OH, optionally substituted C₁-C₆ alkyl and optionally substituted alkoxy; R⁵ and R⁹ are independently selected from selected from H and OH, wherein one of R⁵ and R⁹ is OH; R⁶ is selected from H and an optionally substituted monosaccharide; R⁷ and R⁸ are independently selected from H, OH, optionally substituted C₁-C₆ alkyl and optionally substituted alkoxy; n is an integer selected from 0 and 1; "-----" is selected from a single and a double bond; as well as tautomers, geometrical isomers, optically active forms, pharmaceutically acceptable salts and pharmaceutically active derivative thereof for use in the prevention and/or treatment of a cancer.

2. A compound for use according to claim 1, wherein **R¹** is a lactone of Formula (II): 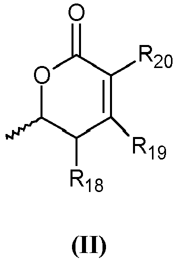 wherein **R¹⁸**, **R¹⁹** and **R²⁰** are independently selected from H and optionally substituted C₁-C₆ alkyl such as methyl.

3. A compound for use according to claim 1, wherein **R¹** is a Formula (III): 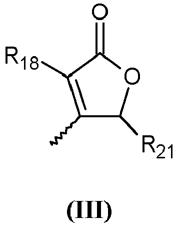 wherein **R¹⁸** and **R²¹** are independently selected from H and optionally substituted C₁-C₆ alkyl.

4. A compound for use according to anyone of claims 1 to 3 wherein **R²** is H.

5. A compound for use according to anyone of claims 1 to 4 wherein **R³** and **R⁴** are optionally substituted C₁-C₆ alkyl.

6. A compound for use according to anyone of claims 1 to 5 wherein **R⁵** is OH.

7. A compound for use according to anyone of claims 1 to 6 wherein **R⁹** is OH.

8. A compound for use according to anyone of claims 1 to 7 wherein **R⁶** is H.

9. A compound for use according to anyone of claims 1 to 7 wherein **R⁶** is a monosaccharide of Formula (IV): 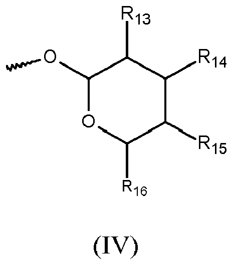 wherein **R¹³, R¹⁴, R¹⁵** and **R¹⁶** are independently selected from H, -OR¹⁷ and optionally substituted C₁-C₆ alkyl and wherein **R¹⁷** is selected from H and optionally substituted C₁-C₆ alkyl, wherein at least one of **R¹³**, **R¹⁴, R¹⁵** and **R¹⁶** is -OR¹⁷.

10. A compound for use according to anyone of claims 1 to 7, wherein R¹⁶ is selected from optionally substituted C₁-C₆ alkyl and -OR¹⁷ wherein **R¹⁷** is optionally substituted C₁-C₆ alkyl.

11. A compound for use according to claim 1, wherein said compound is selected from the following group: 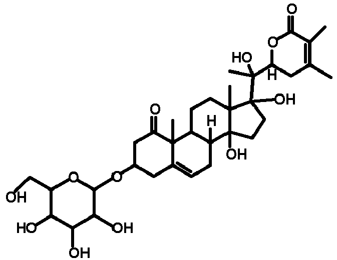 (CAP1, coagulin-L, (14*R*,17S,20*R*,22*R*)-3β,14,17,20-Tetrahydroxy-1-oxo-witha-5,24-dienolide 3-O-β-D-glucopyranoside); 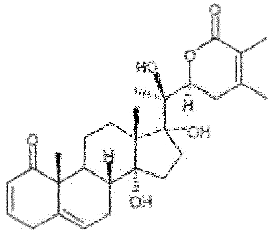 (CAP2, withanolide F, (17α,22R)-14,17,20-Trihydroxy-22,26-epoxyergosta-3,5,24-triene-1,26-dione); and 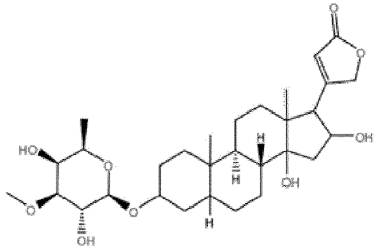 (CAP3, 2,6-dimethyltetrahydropyran-3,4,5-triol; ethane; 3-(3,14,16-trihydroxy-10,13-dimethyl-1,2,3,4,5,6,7,8,9,11,12,15,16,17-tetradecahydro cyclopenta[a]phenanthren-17-yl)-2H-furan-5-one) as well as tautomers, geometrical isomers, optically active forms and pharmaceutically acceptable salts thereof

12. A pharmaceutical formulation comprising a compound of Formula (I) as described in any one of claims 1 to 11, or a tautomer, a geometrical isomer, an optically active form, a pharmaceutically acceptable salt, a pharmaceutically active derivative thereof or a mixture thereof, further combined with at least one co-agent useful in the treatment of a cancer, and at least one pharmaceutically acceptable carrier.

13. A compound for use according to any one of claims 1 to 11, wherein the cancer is a cancer associated with the cancer stem cells.

14. A compound for use according to any one of claims 1 to 13, wherein the cancer is selected from brain cancer, breast cancer, colon cancer, ovary cancer, pancreas cancer, prostate cancer, melanoma cancer, multiple myeloma cancer, skin cancer, oral and neck cancer, head and neck cancer, lung cancer, gastric cancer, mesothelioma, glioblastoma, medulloblastoma or cervical cancer.

15. A compound for use according to any one of claims 1 to 14, wherein the cancer is selected from colon cancer, lung cancer, skin cancer and glioblastoma.


SEQUENCE LISTING

<110>  UNIVERSITE DE GENEVE

<120>  NEW AGENTS FOR MODULATION OF WNT-TCF SIGNALLING AND USES THEREOF

<130>  P1969EP00

<160>  42

<170>  PatentIn version 3.5

<210>  1
<211>  20
<212>  DNA
<213>  Artificial Sequence

<220>
<223>  AXIN2 Fw

<400>  1
ctccttatcg tgtgggcagt                                                    20


<210>  2
<211>  20
<212>  DNA
<213>  Artificial Sequence

<220>
<223>  AXIN2 Rev

<400>  2
ccaactccag cttcagcttt                                                    20


<210>  3
<211>  17
<212>  DNA
<213>  Artificial Sequence

<220>
<223>  ASCL2 Fw

<400>  3
gcgttccgcc tactcgt                                                       17


<210>  4
<211>  16
<212>  DNA
<213>  Artificial Sequence

<220>
<223>  ASCL2 Rev

<400>  4
ggcttccggg gctgag                                                        16


<210>  5
<211>  20
<212>  DNA

<213>  Artificial Sequence

<220>
<223>  LEF1 Fw

<400>  5
aacctctcag gagccctacc                                                    20


<210>  6
<211>  19
<212>  DNA
<213>  Artificial Sequence

<220>
<223>  LEF1 Rev

<400>  6
cacgggcact ttatttgat                                                     19


<210>  7
<211>  20
<212>  DNA
<213>  Artificial Sequence

<220>
<223>  LGR5 Fw

<400>  7
ggagcattca ctggccttta                                                    20


<210>  8
<211>  20
<212>  DNA
<213>  Artificial Sequence

<220>
<223>  LGR5 Rev

<400>  8
ctggacgggg atttctgtta                                                    20


<210>  9
<211>  19
<212>  DNA
<213>  Artificial Sequence

<220>
<223>  cMYC Fw

<400>  9
tggtcttccc ctaccctct                                                     19


<210>  10
<211>  20
<212>  DNA
<213>  Artificial Sequence

<220>

<223>  cMYC Rev

<400>  10
gatccagact ctgacctttt                                                   20


<210>  11
<211>  20
<212>  DNA
<213>  Artificial Sequence

<220>
<223>  CDKN1A/p2 Fw

<400>  11
gactctcagg gtcgaaaacg                                                   20


<210>  12
<211>  20
<212>  DNA
<213>  Artificial Sequence

<220>
<223>  CDKN1A/p21 Rev

<400>  12
aagatgtaga gcgggccttt                                                   20


<210>  13
<211>  20
<212>  DNA
<213>  Artificial Sequence

<220>
<223>  EPHB2 Fw

<400>  13
atgcggaaga ggtggatgta                                                   20


<210>  14
<211>  20
<212>  DNA
<213>  Artificial Sequence

<220>
<223>  EPHB2 Rev

<400>  14
ccttgaaagt cccagatgga                                                   20


<210>  15
<211>  20
<212>  DNA
<213>  Artificial Sequence

<220>
<223>  EPHB3 Fw

<400>  15

tgccactcaa gctctactgc                                                    20

<210>  16
<211>  20
<212>  DNA
<213>  Artificial Sequence

<220>
<223>  EPHB3 Rev

<400>  16
gttattgtgg caggtgcaga                                                    20

<210>  17
<211>  20
<212>  DNA
<213>  Artificial Sequence

<220>
<223>  SOX4 Fw

<400>  17
aaaccaacaa tgccgagaac                                                    20

<210>  18
<211>  20
<212>  DNA
<213>  Artificial Sequence

<220>
<223>  SOX4 Rev

<400>  18
gttcatgggt cgcttgatgt                                                    20

<210>  19
<211>  19
<212>  DNA
<213>  Artificial Sequence

<220>
<223>  ZNRF3 Fw

<400>  19
cgggtcatcc cctgtactc                                                     19

<210>  20
<211>  20
<212>  DNA
<213>  Artificial Sequence

<220>
<223>  ZNRF3 Rev

<400>  20
acgtgagagg ttgctggtct                                                    20

<210>  21
<211>  23
<212>  DNA
<213>  Artificial Sequence

<220>
<223>  KITLG Fw

<400>  21
ttccatcata gacaaacttg tga                                               23


<210>  22
<211>  20
<212>  DNA
<213>  Artificial Sequence

<220>
<223>  KITLG Rev

<400>  22
gtaaagagcc tgggttctgg                                                   20


<210>  23
<211>  19
<212>  DNA
<213>  Artificial Sequence

<220>
<223>  CXCL3 Fw

<400>  23
cgtccgtggt cactgaact                                                    19


<210>  24
<211>  18
<212>  DNA
<213>  Artificial Sequence

<220>
<223>  CXCL3 Rev

<400>  24
atgacttcgg tttgggcg                                                     18


<210>  25
<211>  20
<212>  DNA
<213>  Artificial Sequence

<220>
<223>  IL33 Fw

<400>  25
tgagtctcaa cacccctcaa                                                   20


<210>  26
<211>  20
<212>  DNA

<213>  Artificial Sequence

<220>
<223>  IL33 Rev

<400>  26
gttggcatgc aaccagaagt                                                   20


<210>  27
<211>  19
<212>  DNA
<213>  Artificial Sequence

<220>
<223>  ID3 Fw

<400>  27
accttcccat ccagacagc                                                    19


<210>  28
<211>  18
<212>  DNA
<213>  Artificial Sequence

<220>
<223>  ID3 Rev

<400>  28
cttccggcag gagaggtt                                                     18


<210>  29
<211>  20
<212>  DNA
<213>  Artificial Sequence

<220>
<223>  BMP4 Fw

<400>  29
gatccacagc actggtcttg                                                   20


<210>  30
<211>  20
<212>  DNA
<213>  Artificial Sequence

<220>
<223>  BMP4 Rev

<400>  30
gggatgctgc tgaggttaaa                                                   20


<210>  31
<211>  21
<212>  DNA
<213>  Artificial Sequence

<220>

<223>  ZMYND8 Fw

<400>  31
cgagacccag agtaaagcca t                                                21


<210>  32
<211>  21
<212>  DNA
<213>  Artificial Sequence

<220>
<223>  ZMYND8 Rev

<400>  32
gatgtatccg catagtcagg g                                                21


<210>  33
<211>  21
<212>  DNA
<213>  Artificial Sequence

<220>
<223>  EED Fw

<400>  33
gtgacgagaa cagcaatcca g                                                21


<210>  34
<211>  21
<212>  DNA
<213>  Artificial Sequence

<220>
<223>  EED Rev

<400>  34
tatcagggcg ttcagtgttt g                                                21


<210>  35
<211>  21
<212>  DNA
<213>  Artificial Sequence

<220>
<223>  SMARCAL1 Fw

<400>  35
acagcatcag aggactagct c                                                21


<210>  36
<211>  21
<212>  DNA
<213>  Artificial Sequence

<220>
<223>  SMARCAL1 Rev

<400>  36

cactggctta caagactccc t                                                21

<210>  37
<211>  20
<212>  DNA
<213>  Artificial Sequence

<220>
<223>  TBL1XR1 Fw

<400>  37
ccagttccca tttcctgcca                                                  20

<210>  38
<211>  21
<212>  DNA
<213>  Artificial Sequence

<220>
<223>  TBL1XR1 Rev

<400>  38
acaccacaaa aggaggcact t                                                21

<210>  39
<211>  20
<212>  DNA
<213>  Artificial Sequence

<220>
<223>  SETD6 Fw

<400>  39
ggagggaaca gcaagcctta                                                  20

<210>  40
<211>  20
<212>  DNA
<213>  Artificial Sequence

<220>
<223>  SETD6 Rev

<400>  40
tcagggaaca gggaaactgc                                                  20

<210>  41
<211>  20
<212>  DNA
<213>  Artificial Sequence

<220>
<223>  TAF5L Fw

<400>  41
caggcagaac cccagcaata                                                  20

<210>  42
<211>  20
<212>  DNA
<213>  Artificial Sequence

<220>
<223>  TAF5L Rev

<400>  42
cactgtgctc ttcggactgt                                                    20